(19) **Europäisches Patentamt / European Patent Office / Office européen des brevets**

(11) **EP 2 386 250 B1**

(12) **EUROPEAN PATENT SPECIFICATION**

(45) Date of publication and mention
of the grant of the patent:
**11.09.2013 Bulletin 2013/37**

(21) Application number: **11173441.4**

(22) Date of filing: **25.10.2007**

(51) Int Cl.:
*A61B 8/06* (2006.01)　　*A61B 8/08* (2006.01)
*G01S 15/58* (2006.01)　　*G01S 7/52* (2006.01)
*A61B 5/0456* (2006.01)　　*A61B 5/107* (2006.01)
*A61B 5/021* (2006.01)

(54) **Cardiac parameter measurement**

Herzparametermessung

Mesure de paramètres cardiaques

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **26.10.2006 US 863002 P**

(43) Date of publication of application:
**16.11.2011 Bulletin 2011/46**

(62) Document number(s) of the earlier application(s) in
accordance with Art. 76 EPC:
**07827260.6 / 2 079 368**

(73) Proprietor: **Cardiogal Ltd.**
**69395 Tel-Aviv (IL)**

(72) Inventors:
• **Sharf, Yehuda**
**69395 Tel-Aviv (IL)**

• **Lancaster, Gilead I.**
**Redding, CT Connecticut 06896 (US)**

(74) Representative: **Becker Kurig Straus**
**Bavariastrasse 7**
**80336 München (DE)**

(56) References cited:
**EP-A- 0 747 010　　EP-A- 1 273 267
US-A- 4 233 989　　US-A- 5 409 010
US-A- 5 868 676**

• **WALLENTIN GURON ET AL: "The E/e filling index
and right ventricular pressure in relation to
applied international Doppler recommendations
of left ventricular filling assessment",
EUROPEAN JOURNAL OF
ECHOCARDIOGRAPHY, HARCOURT
PUBLISHERS, EDINBURGH, GB, vol. 6, no. 6,
December 2005 (2005-12), pages 419-428,
XP005156963, ISSN: 1525-2167**

## Description

The present invention, in some embodiments thereof, relates to non-invasive measurement of cardiac parameters, for example, measurement of cardiac output.

## BACKGROUND

The Swan Ganz catheter is the standard method for measuring heart pressures and cardiac function in the intensive care unit and after various types of surgery (particularly open-heart surgery). Patients recovering from cardiac surgery and those admitted to Intensive Care Units (ICU's) with heart failure often require hemodynamic (blood flow and pressure) monitoring which usually involves a Swan-Ganz catheter that requires an invasive procedure for placement. The Swan-Ganz catheter (SG) is used to assess the "fluid status" by measuring the pressures of the left ventricle (main pumping chamber of the heart) and the pulmonary artery. If the heart pressures are low, the patient often requires more fluid; and if the pressures are high then fluid must be restricted and even expelled (e.g. by giving medication to increase urination). The catheter is often used for longer periods of time (up to 1-2 days) to assess the effectiveness of the therapy. In addition, the SG catheter can measure the cardiac output (i.e. the amount of blood that the heart pumps per minute) and indirectly derive the contractility of the heart muscle. These parameters can be crucial for determining the clinical treatment, in some situations.

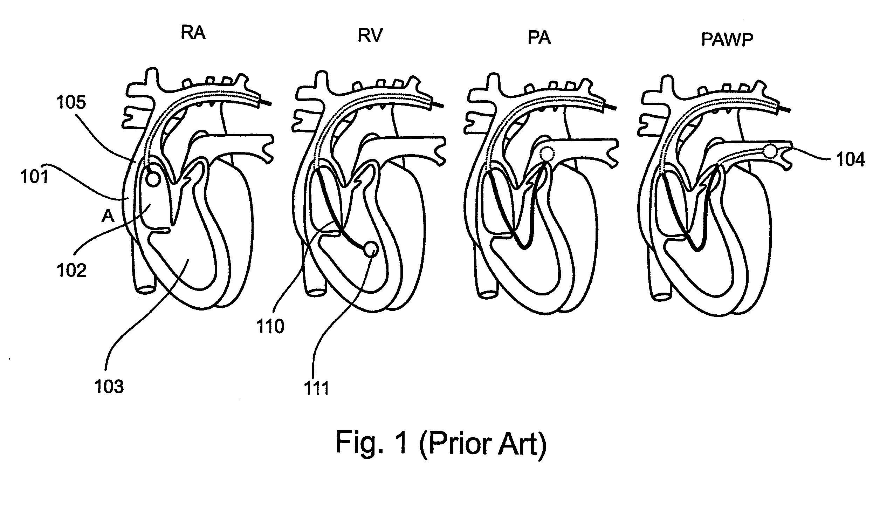

Fig. 1 illustrates the insertion procedure of central catheter of a prior art hemodynamic monitor. A Swan Ganz (SG) catheter 110 is placed through a vein 105 and wound up, in the direction of blood flow, through a right atrium 102 and through a right ventricle 103. The tip ultimately rests in one of the main pulmonary arteries 104. When a balloon 111 at the end of the SG is not inflated (when it is "down"), a hole at the tip of the catheter is exposed to the pressure of the pulmonary artery. Most of this pressure is pressure coming from the right ventricle when it contracts and the "pulmonary vascular resistance" which generally depends on pulmonary vascular tone and pressures from the left side of the heart. The pulmonary artery is connected to the left side of the heart through a large number of arterioles and capillaries that connect to the pulmonary veins. The pulmonary veins are connected directly to the left atrium and the left atrium is connected to the left ventricle during diastole when the mitral valve is open.

When balloon 111 is inflated ("up") the effects of the RV (right ventricle) contractions and the vascular reaction to the RV contractions downstream of the balloon are virtually eliminated. The opening at the end of the SG is therefore only affected by the transmittal of pressures from the left side of the heart. This is called "wedge pressure". In systole, when the mitral valve is closed this "wedge pressure" is equal to the left atrial pressures. When the valve is open in diastole the pressure is equal to the left ventricular pressure. The "wedge pressure" is used as a measurement of LV diastolic pressures. These parameters and assumptions have been used for years and considered the standard of care for measuring these left ventricular pressures in the intensive care unit.

In some cases, it is desirable to use a non-invasive approach, for example, to avoid infection risks typically associated with long-term catheterization.

At present the typical non-invasive method for making these measurements is a high-end echocardiograph machine specially equipped with tissue Doppler imaging. Echocardiography is the gold standard imaging technique for detecting mechanical heart disease in the clinical setting. It is used to measure ejection fraction, dimensions, thickness and movement of the ventricles. The "Doppler Effect" has been used in echocardiography for many years, in which frequency shifts (i.e. Doppler Effect) of ultrasound waves are used to calculate blood flow speeds and direction in the heart chambers. Tissue Doppler echocardiography (TDE) is a relatively recent addition to the diagnostic ultrasound examination; it permits an assessment of heart muscle motion using Doppler shifts. Heart muscle motion is characterized by low velocity (of the order of 0.1 meter/ second) relative to the velocity of blood flow (typically of the order of 1 meter/ second) , and by much higher intensity of echo amplitude (+40dB) . "Recommendations for Quantification of Doppler Echocardiography: A Report From the Doppler Quantification Task Force of the Nomenclature and Standards Committee of the American Society of Echocardiography", by Miguel A. Quinones, Catherine M. Otto, Marcus Stoddard, Alan Waggoner, and William A. Zoghbi (J Am Soc Echocardiogr 2002; 15: 167- 84) , provides various definitions for Doppler Echocardiography, which may be useful in the practice of some embodiments of the invention.

Doppler techniques measure the motion of blood and muscle tissues. The Doppler technique allows the quantitative assessment of heart muscle contraction and relaxation speeds. In addition, the Doppler technique has greater temporal resolution (ability to detect rapidly occurring events) than standard 2-dimensional echocardiography. This feature allows for more accurate "timing" of various events in normal (and abnormal) heart contraction and relaxation.

EP 0 747 010 to Schmiesing et al. teaches a technique for continuously determining and displaying the peak velocities of spectral Doppler information.

Standard transmitral flow velocity profiles have been shown to be very dependent on loading pressures; "preload" is the pressure pushing the blood through the mitral valve (i.e. left atrial pressure). This effect of preload is particularly important in the E wave velocity (Doppler flow wave corresponding to early diastolic filling of the LV in diastole). The higher the preload (i.e. left atrial diastolic pressure), the higher the E wave velocity. It has also been shown that E' (tissue Doppler wave corresponding to early diastolic relaxation of the LV (left ventricle)) is relatively load independent and that the ratio of E/E' is well correlated with the left ventricular diastolic pressures (LVDP). S' (tissue Doppler wave corresponding to LV systolic contraction) measures the strength of myocardial contraction and can be used to assess myocardial function during systole.

The concept that Doppler ultrasound is capable of assessing pressures is well accepted in cardiology. Specifically, the concept is most accepted in the evaluation of pulmonary artery pressures.

The explanation for the relation between measurement of velocities and pressure follows:

The velocity (V) of the tricuspid regurgitant jet (tricuspid regurgitation is present in most people to some degree), is related to the pressure difference (ΔP) between the right ventricle (RV) and the right atrium (RA) by the equation:

$$\Delta P = 4V^2$$

If the right atrial pressure is known, it can be summed with ΔP to yield the right ventricular systolic pressure in mmHg). Most cardiologists use 10mmHg as an estimate of the RA pressure, but may increase the estimate if there is inferior vena caval dilatation. In systole, the pulmonic valve is open to the pulmonary artery (PA), so that RV systolic pressure and PA systolic pressures are the same. Consequently PA systolic pressure is considered as being equal to $4V^2+10$. These measurements have had good clinical correlation with SG catheter measurements and are used in almost every ultrasonic echo procedure performed.

The derivation of LV diastolic pressures is somewhat more involved, but also depends on Doppler velocities:

First, Doppler velocity of the rapid filling phase of diastole is reflected in the E wave of mitral inflow. The velocity of the E wave is dependant on the pressure differences between the LA and LV when the mitral valve opens in the beginning of diastole. Consequently the E velocity can be increased by increasing "preload", (i.e. more fluid pressure from the venous side) .

On the other hand, tissue Doppler velocities of early diastole (E') reflect the passive and active relaxation of the left ventricular muscle. The passive relaxation is due to the force of the early filling of blood in the ventricle. The active component is determined by the compliance of the ventricle. In practice E' appears to be mostly due to the active component and seems to be relatively load independent.

Since E is a relatively preload dependant measure and E' relatively load independent, the velocity ratio E/E' reflects the "loading pressure" of the LV during diastole. It has been reported that E/E' correlates well with measurements using catheters in the Left ventricle. This has been studied in several clinical settings, particularly in the assessment of LV diastolic function and the estimation of left ventricular filling pressures (Circulation 2004; 109:2432-2439, JACC 2006; 47:1891-1900). Additional papers describing measurements in the heart are Alain Combes et al., "Tissue Doppler imaging estimation of pulmonary artery occlusion pressure in ICU patients", Intensive Care Med (2004) 30:75-81 and Cheuk-Man Yu, John E. Sanderson, Thomas H. Marwick, and Jae K. Oh, "Tissue Doppler Imaging: A New Prognosticator for Cardiovascular Diseases", J. Am. Coll. Cardiol. 2007;49;1903-1914. More specifically, it has been reported that E/E' of ≥15 correlates with Left ventricular diastolic pressures of ≥15 mmHg. SG derived pressures of 15 mmHg are considered the border between normal (<15) and abnormal (> 15) conditions. Even during stress testing it has been found that when the LV diastolic pressures change with exercise (as measured with a catheter in the LV), the E/E' ratio changes accordingly, with excellent correlation.

Other parameters that have been used to reflect diastolic function have included the E/A velocity ratio (where E is the velocity of early rapid filling and A is the velocity of the atrial contraction) and A' (end diastolic atrial contraction tissue) velocity. The deceleration slope of the E wave has also been correlated with restrictive diastolic pathology. Additional parameters are E wave deceleration, inflow VTI (sometimes used instead of outflow VTI) and A-wave duration which may reflect an AV delay.

Doppler ultrasound is also useful in the assessment of systolic function of the LV. An important parameter of heart function is the amount of blood ejected out of the heart into the aorta during each contraction. This is called the stroke volume or SV. If the Doppler beam is directed parallel to the flow of blood, the integration over time of instantaneous blood flow velocity will give the average velocity of blood over time. This is called the velocity time integral (VTI). Multiplying the VTI during a cardiac cycle by the cross-sectional area of the vessel through which the blood traverses (or in the case of the left ventricle, the narrowing just before the aortic valve called the left ventricular outflow tract, LVOT) will give a measurement of stroke volume (SV). This measurement has been well validated in many studies and is commonly used in most echo laboratories.

Cardiac output (CO) is another parameter for assessing LV systolic function and is defined as the amount of blood expelled per unit time (usually per minute). With SG, the CO is commonly derived by "thermodilution" whereby saline is injected into the right side of the heart and the rate of temperature change determines how much blood flow has occurred. SV is then derived by dividing CO by the simultaneous heart rate. This derivation may be inaccurate, especially in cases where there is tri-cuspid or mitral valve disease or in the presence of atrial fibrillation and frequent extrasystoles (early beats).

Doppler derived SV is relatively direct. It is found by multiplying the measured VTI of the LVOT (and aortic valve) by the LVOT area. It is therefore less dependant on mitral disease or the rhythm.

For systolic functional assessment, tissue Doppler derived S' has been shown to correlate with LV systolic function. Studies have shown that S' correlates with LV ejection fraction (the fraction of blood that is expelled from the heart during systole) and has been shown to help assess global contraction function of the left ventricle in heart failure (JASE 2005; 18:148-154, and also in the JACC review article, above).

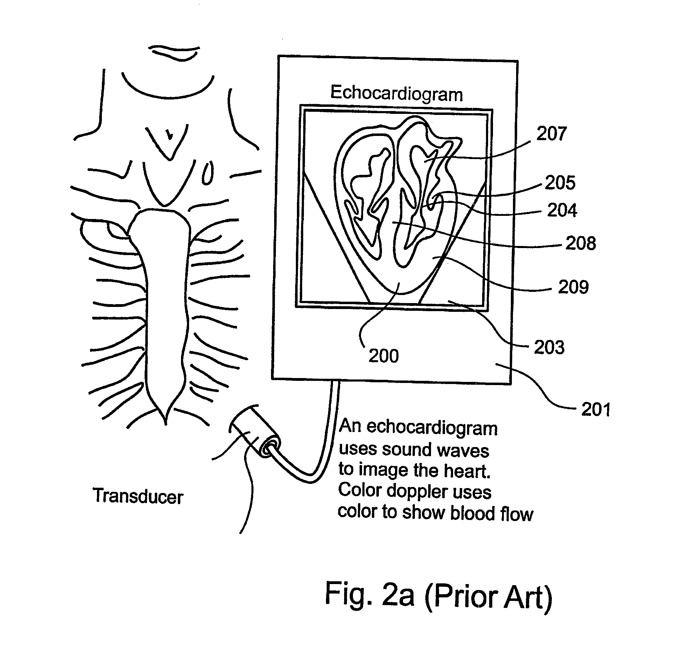

Fig. 2a illustrates a modem ultrasound scanner 201 with a probe placed externally 202 for the apical 4-chamber view of the heart 200. The monitor displays 203 color coded Doppler velocities superimposed on a 2D anatomical image of the heart.

In both echocardiography and Doppler studies the positioning of ultrasound transducer is considered to be of great importance. Most of the published data on mitral valve inflow and tissue Doppler in humans use apical views because this is considered to be the correct way to look at transmitral inflow velocities 204. The apical views are also considered best for reliable measurement of the longitudinal muscle velocity of the left ventricle. These apical tissue Doppler measurements, usually performed at the level of the mitral valve annulus, isolate only the longitudinal muscle contractions in systole (for S') or relaxation in diastole (for E' and A') of the left ventricle measurements.

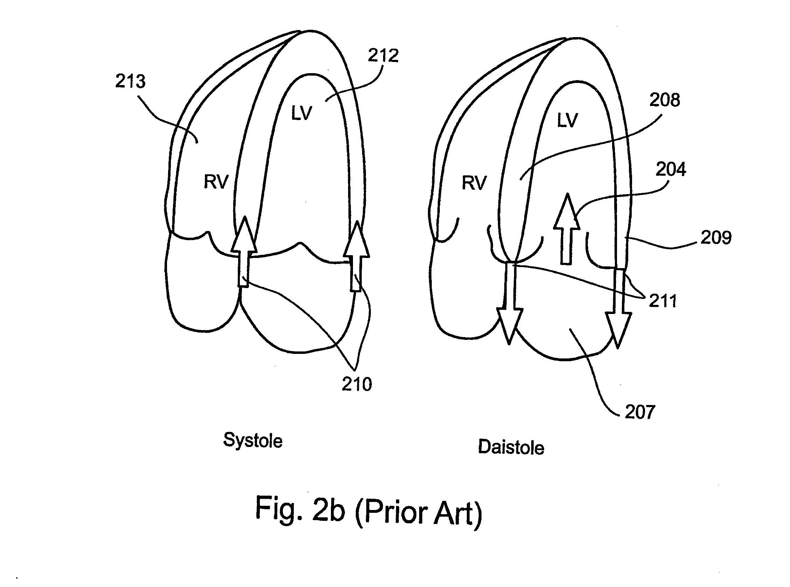

Fig. 2b shows the heart as seen in the apical 4-chamber view in systole and in diastole. In systole, a base of the heart, 210 (i.e. the myocardium closest to the mitral valve annulus), travels up toward the apex (as well as apical ultrasound transducer 202). In diastole, the base of the heart travels away from the apex and transducer 202 as the myocardium relaxes (211). At the same time, the mitral valve opens and the blood in a left atrium, 207, flows rapidly, 204, into a left ventricle (LV) 212.

Another important technical consideration is placement of the sample (i.e., where the Doppler signal will be measured from). For transmitral inflow 204 the usual sample placement is a "pulsed" Doppler at the tip of the mitral valve 205 leaflets and the tissue Doppler sample is placed on either the septal wall 208 or lateral wall 209 at the level of the mitral valve annulus. On standard echo/Doppler systems sample placement is very angle dependant and often requires several attempts to obtain the optimal position of the probe.

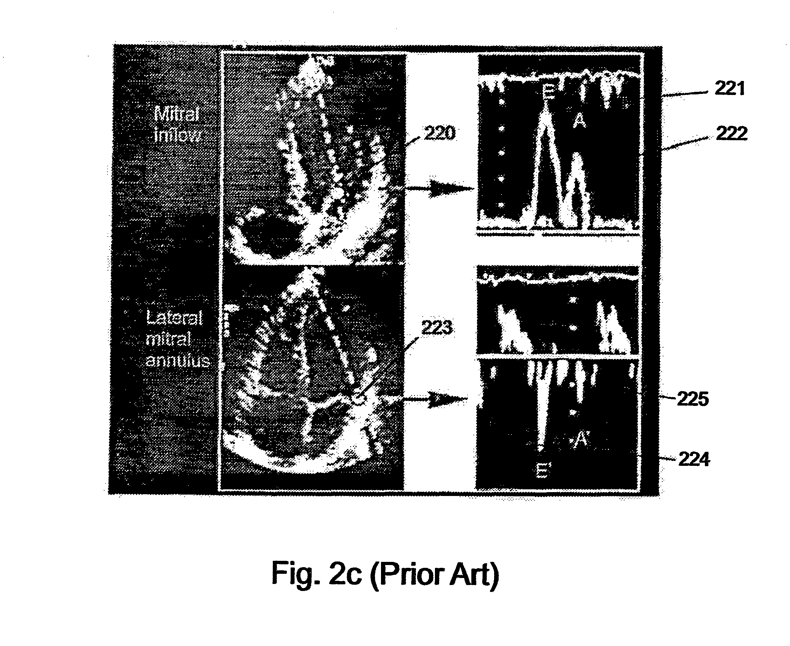

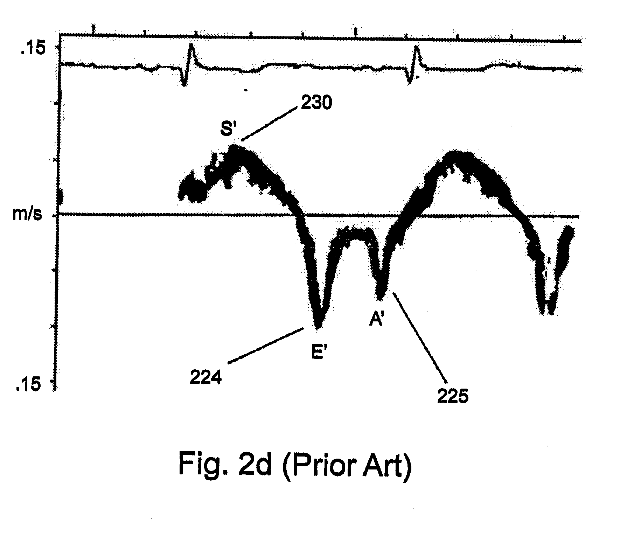

Fig. 2c shows examples of transmitral inflow sampling, 220, and the resultant spectral image obtained (recorded as velocity over time). The components of transmitral inflow are marked as E (221) and A (222). It should be noted that the flow is toward the apex and so is toward the transducer 202. The bottom panel shows lateral wall mitral annulus tissue Doppler sampling, 223, and the resultant spectral image with E' (224) and A' (225). In this case the base is moving away from the apex and away from the transducer 202. In systole, a signal S' (230) is toward the transducer 202 giving the resulting typical tracing seen in Fig. 2d.

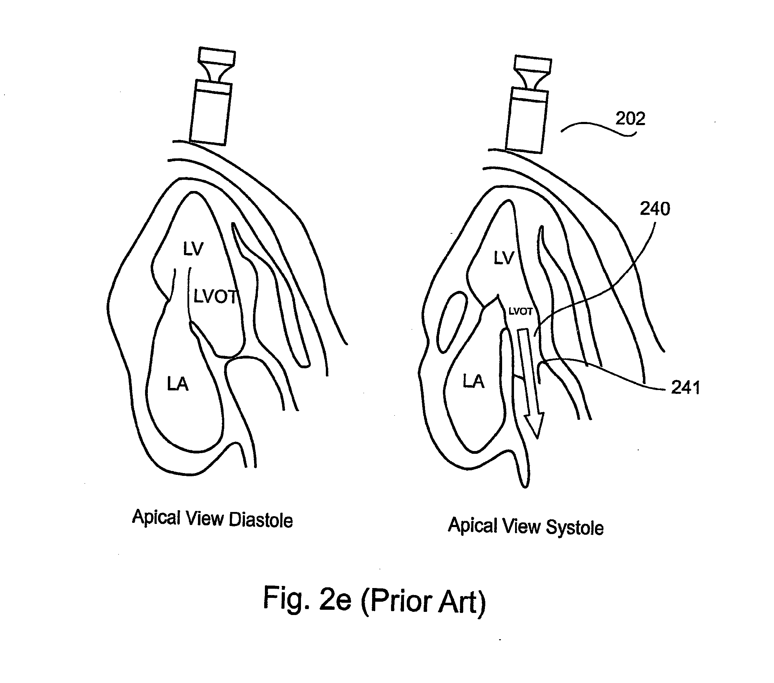

Fig. 2e shows another apical view known as an apical long-axis view. This shows the optimal visualization of the LVOT from the apical orientation. Doppler flow 240 through the LVOT in systole can be measured as it exits the heart through the opened aortic valve 241.

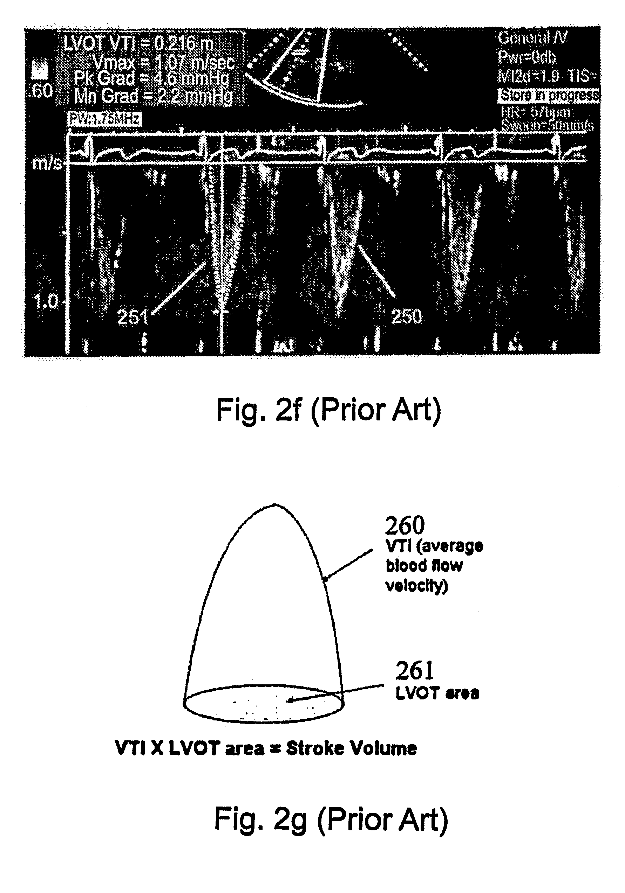

Fig. 2f shows a spectral image of LVOT velocity over time 250. By tracing the "spectral envelope" 251 of the LVOT flow and integrating over the time, the velocity-time-integral (VTI) is derived. The VTI represents the average flow velocity of the blood in the LVOT during systole.

As represented in Fig. 2g, VTI 260 is then multiplied by a LVOT area 261 to yield stroke volume (SV). Most present day echo systems have analysis "packages" that allow the automatic calculation of VTI once the envelope is traced. Typically, such packages require manual tracing of LVOT velocity envelope 251, making it difficult to use this measurement in a clinical monitoring setting.

US Patent No. 7,054,679 to Hirsh discloses a method and a system for monitoring cardiac function and hemodynamics including the left ventricular end diastolic pressure. It teaches constructing a conversion function in a form of a table for translating plurality of non-invasively measured cardiac parameters based on blood flow into their invasive analogous.

US Patent No. 7,043,292 to Tarjan, et al. discloses a method and system for mapping the cardiac electrical activity over an acquired image to show how the signals are propagated along the muscle.

US patent No. 7,022,077 to Mourad et al. and its continuation in part Patent application publication 20040059220 disclose Systems and methods for noninvasive assessment of cardiac tissue properties and cardiac parameters using ultrasound techniques.

US Patent No. 6, 875, 176 to Mourad et al. discloses a system for collecting acoustic data and making determination of induced and/or intrinsic tissue displacement (s), using one or more transducers mechanically focused on a target tissue.

US Patent No. 5,409,010 to Beach and Overbeck discloses an ultrasonic pulse-echo medical device for determining the angular heading and magnitude of the velocity of blood flowing through a blood vessel in the heart.

US patents No. 5,188,106 to Nappholz, et al. and No. 5,139,020 to Koestner, et al. disclose methods of operating a hemodynamic control apparatus for determining a patient's hemodynamic status and to regulate hemodynamic therapy, comprising ultrasonically monitoring of at least one parameter corresponding to the volume of blood flow per unit of time within the patient's cardiovascular system., using an invasive device.

US patent 6261233 to Kantorovich describes a method of detecting a blood flow vector direction in an Aorta.

US patent 6730030 to Palti describes detecting a coronary stenosis using blood velocity measurements.

## SUMMARY OF THE INVENTION

The invention is set out in independent claims 1 and 8.

Embodiments not identified as embodiments of the invention do not fall under the scope of claims 1 and 8.

A broad aspect of some embodiments of the invention relates to detecting and/or measuring locations of interest in the heart using non-imaging methods. In an exemplary embodiment of the invention, once such locations are identified, a non-imaging signal is used to acquire information useful in assessing one or more cardiac parameters. In an exemplary embodiment of the invention, one or more transducers for such detection are aimed automatically and/or their signals processed so that placing the transducers and/or maintaining them in proper aim does not require expert ability and/or, in some embodiments, an imaging system.

Exemplary embodiments of the invention take advantage of the realization that at times where there is a signal of interest (to those embodiments) the signal is a peak signal which is substantially unique in the heart (above a threshold value), for example, peak tissue movement and peak blood flow (at the beginning of the burst). In an exemplary embodiments of the invention, the extraction methods utilize this insight and provide useful information because most of the (maximum) flow of interest is concentrated in that peak.

In an exemplary embodiment of the invention, the locations are based on a signature behavior. In one example, maximum tissue motion in a certain general direction and/or maximum blood flow in a certain general direct, and/or such motions coupled with an indication of cardiac cycle, are sufficient to identify the location in the heart providing the signal. In another example, a signature of a mitral vale or other valve is used as a landmark and/or for verification. For example, when in an apical view, the mitral valve signature indicates a plane to look for tissue motion in and a distance to look for blood motion in.

In an exemplary embodiment of the invention, the maximum values are spatial extreme values alternatively or additionally to being temporal extreme values.

In an exemplary embodiment of the invention, a non-focused beam is used for such extractions.

In an exemplary embodiment of the invention, the locations/tissue/structure that are identified include one or both of tissue at the mitral valve and/or an initial bolus of blood after the mitral valve opens.

In an exemplary embodiment of the invention, use is made of the fact that for some applications, there is only one peak value at any given time and that peak value is sufficient for clinically useful measurement. Optionally, the tissue generating the peak value changes over time. In some cases, two peak values are found, one for blood and one for muscle, substantially simultaneously. As used herein, the term "muscle" is used to relate to tissue in the heart which is not blood, and includes also valvular tissue and blood vessels. However, as most of the heart is formed of muscle, this general term is used in an encompassing manner.

In an exemplary embodiment of the invention, one or more of the following are measured: e'=early diastolic tissue movement; a'=late diastolic tissue movement; s' = systolic tissue movement; e=early diastolic transmitral flow; and/or a=later diastolic transmitral flow. In an exemplary embodiment of the invention, the acquired data is used to calculate a cardiac output (e.g., based on VTI, velocity time integral, used for calculating stroke volume).

In an exemplary embodiment of the invention, the non-imaging means is a broad angle non-imaging an ultrasonic sensor. In an exemplary embodiment of the invention, a signal from a structure of relevance is extracted from other parts of a signal, based on a behavior of the signal.

In an exemplary embodiment of the invention, a plurality of transducers are used and the system selects which transducers to use together to obtain a useful signal. Optionally, such selection is repeated, for example, periodically or as needed.

In an exemplary embodiment of the invention, one or more acoustic transducers are placed on a patient's chest and acquire signals from the heart. Optionally, one or more of the transducers are aimed generally at the area of the mitral valve. In an exemplary embodiment of the invention, a single transducer is used and it is aimed from an axial view.

In an exemplary embodiment of the invention, a cardiac parameter determination system does not need to be aimed with precision, rather the processing of the signals allowed the correct structures to be sensed.

In an exemplary embodiment of the invention, the system is used for continuous monitoring, for example, on a beat-by-beat level. It is hypothesized that conventional E/E' measurement can be false in case of atrial fibrillation. In an exemplary embodiment of the invention, a beat to beat calculation of the ratio E/E' overcomes this problem (and/or problems due to beat-to-beat variation of other types), at least for non-fibrillating cycles, or as an average over time of what happens in each of a plurality cycle.

It should be noted that in some embodiments of the invention, the measurement is useful because it is not only non-invasive but also does not interfere with the hemodynamics being measured and/or with a patient's body functions.

In an exemplary embodiment of the invention, only a single transducer (or only one receiver, or with a separate transmitter) is used. Optionally, the exact angle of incidence at the target tissue is not critical, by using a measure which is a ratio between two measured parameters (e.g., tissue motion in one direction and blood in an opposite direction), so that an effect of the angle on one is close to the effect of the angle on the other one, at least for relatively small angles (e.g., cosine of the angle between the direction of blood and line of sight <0.75, and cosine of the angle between the direction of blood and direction of tissue < 0.93). In addition, in an exemplary embodiment of the invention, what is measured is maximum velocity, which generally indicates the same tissue location, even if the detector aim is not aligned with the velocity direction.

It should be noted that a major limitation of the Doppler technique on standard echocardiography is that it is extremely angle dependant. Small deviations of angles may lead to significant underestimation of Doppler velocities and therefore reduce the reliability of the resultant measurements.

In an exemplary embodiment of the invention, multiple detectors are used, for example three detectors, with one transmitter. Optionally, all transducers are transmitters and receivers and the system selects which ones to use as transmitters and which to use as receivers. Optionally, a velocity vector is reconstructed from the signals of the three detectors, for example, using geometric considerations to reconstruct a vector from three projections thereof.

In an exemplary embodiment of the invention, the detectors are broad-beam detectors and each includes signal contributions from various parts of the heart. In an exemplary embodiment of the invention, what is extracted is not an actual velocity vector, but a general indication of scaling (e.g., between tissue and blood) and direction. Optionally, it is assumed that the signal of interest is a peak (optionally with a minimum width, or other restrictions on the morphology may be used) shape and these shapes are aligned, even if they are not aligned in time or space. It should be noted that different components of the velocity vector may arise from different parts of a tissue being measured, however, as a peak of a tissue unit is being looked at the results are useful. In an exemplary embodiment of the invention, the peak is basically indicative of a single maximum vector, during a time where any turbulent motion is significantly smaller (e.g., less than 50%, less than 70%), so discrimination is easier. Optionally, what is extracted is a measure indicative of a movement vector of a bolus of blood and/or muscle tissue.

In an exemplary embodiment of the invention, the signal is analyzed to determine the timing of various events in the heart, from a functional perspective, for example, mitral valve opening and aortic valve opening (e.g., for identifying LVOT).

In an exemplary embodiment of the invention, a non-imaging mode using multiple transducers uses Doppler signals to reconstruct a portion of the heart without using an imaging sensor. In an exemplary embodiment of the invention, the methods described herein are used to detect peak velocities of various parts of the heart, localize them in space and thereby generate a structural map. Optionally, such a map is used to assist in aiming a beam for analysis of various parameters as described herein. Optionally, for such imaging, no blood signal is analyzed. Optionally, the reconstruction is a functional reconstruction showing how each part moves, possibly at the expense of exact structural detail. A functional image may be acquired, in some cases, with one sensor, however, orientation in space may require user input (e.g., which side is left and which is right).

A particular feature of some embodiments of the invention is that soft tissue parameters and blood flow parameters related to the tissue parameters are measured substantially simultaneously and in a same cycle of the heart. In an exemplary embodiment of the invention, simultaneous measure means that beat-to-beat variations (e.g., due to arrhythmia) do not affect an in-beat measurement. In some cases, one parameter will be taken from one beat and one form the next.

There is provided in accordance with an exemplary embodiment, a method of non-imaging extraction of a value of at least one cardiac parameter, comprising:

a) aiming an ultrasonic sensor in a non-imaging mode and acquiring at least one Doppler signal therefrom;
b) detecting a pattern in said signal, which pattern is associated with a known body portion in the heart; and
c) processing said signal based on said pattern to extract said value,

wherein said pattern comprises an indication of an extreme velocity.

In an exemplary embodiment, said pattern is associated with a behavior of said structure. Optionally, said behavior comprises motion.

In an exemplary embodiment, said portion comprises blood.

In an exemplary embodiment, said portion comprises muscle.

In an exemplary embodiment, said portion is a portion of said heart including blood smaller than 10% by volume thereof.

In an exemplary embodiment, said processing comprises determining both muscle tissue movement and blood movement. Optionally, said determining comprises determining said blood and tissue movements in a same cardiac cycle.

In an exemplary embodiment, said value comprises one or both of E and E'.

In an exemplary embodiment, the method comprises generating an indication of the signal is not usable.

In an exemplary embodiment, aiming comprises aiming in an apical view. Optionally, said signal comprises only a single Doppler signal from a single transmitter.

In an exemplary embodiment, aiming comprises aiming in a direction of maximum motion of said portion, at an angular offset of between 15 and 40 degrees between an aim of the sensor and a vector of the motion.

Optionally, said processing compensates for an angular offset by taking a ratio between measurements of different portions.

In an exemplary embodiment, the method comprises:

positioning said at least one sensor using at most external body markers as a guide; and
wherein said processing comprises processing said signal to determine a portion generated or modulated by a portion of interest of the heart.

In an exemplary embodiment, the method comprises:

acquiring a plurality of simultaneous signals; and
wherein said processing comprises extracting from said signals a vectoric indication of motion of tissue in the heart.

Optionally, said extraction comprises a 3D reconstruction.

In an exemplary embodiment of the invention, said extracting comprises correlating said signals based on an assumption that the extreme value is unique at a given time.

In an exemplary embodiment, said non-imaging sensor has a beam width angle of at least 20 degrees.

In an exemplary embodiment, said processing comprises extracting a relative timing of at least two mechanical events in the heart.

In an exemplary embodiment of the invention, the method comprises continuously monitoring said parameter for at least 15 minutes.

There is provided in accordance with an exemplary embodiment, a method of acquiring signals form a heart, comprising:

(a) positioning a plurality of ultrasonic sensors around a heart and operated in a non-imaging mode;
(b) acquiring signals using said sensors;
(c) reconstructing at least one vector using said probes for a plurality of subsets of said sensors; and
(d) selecting a subset to use, based on a quality of said vector.

In an exemplary embodiment, the method comprises using said subset for a plurality of cycles and then repeating (b)-(d) to determine a new subset.

There is provided in accordance with an exemplary embodiment of the invention, a method of measuring a cardiac parameter, comprising:

a) aiming a non-imaging sensor at a heart and acquiring at least one Doppler signal therefrom;
b) automatically detecting a muscle based signal portion in the signal;
c) automatically detecting a blood flow based signal portion in the signal;
d) processing said detected signal portions to guide an extraction of a value of said parameter.

Optionally, said value is E/E'.

In an exemplary embodiment of the invention, said signal portions are selected according to one or more of time, space and ECG signals.

In an exemplary embodiment, said signal portions each represent an extent of tissue smaller than 10% by volume of cardiac tissue which generates or modulates said signal.

There is provided in accordance with an exemplary embodiment, a method of determining a direction and scale of movement of target tissue, comprising:

(a) acquiring a plurality of Doppler signals from a plurality of different directions of view, of said target tissue; and
(b) using a majority function to select components for use in reconstructing a vector describing motion of said target tissue.

In an exemplary embodiment, combining comprises aligning maximum signal values of said signals, based on an assumption for a shape of said signal.

There is provided in accordance with an exemplary embodiment, apparatus for determining a value of a cardiac parameter, comprising:

(a) a detector adapted to provide a non-imaging ultrasonic signal;
(b) circuitry configured to analyze said signal and determine at least one tissue signature; and
(c) circuitry configured to extract said value from said signature.

Optionally, said a detector comprises a plurality of detectors distributed in space and synchronized in time.

Optionally, said circuitry uses pulsed Doppler signals.

There is provided in accordance with an exemplary embodiment, a method of cardiac monitoring, comprising:

(a) aiming, with a low angular precision, a non-imaging sensor at said heart;
(b) automatically identifying a relevant tissue in said heart by said sensor; and
(c) extracting of a value of a cardiac parameter using a signal detected by said sensor.

There is provided in accordance with an exemplary embodiment, a method of detecting a vector inside a heart, comprising:

(a) acquiring a plurality of Doppler signals from the heart; and
(b) reconstructing a motion vector from said signals at a time where only a single peak motion vector is expected.

There is provided in accordance with an exemplary embodiment, a method of tissue imaging, comprising:

(a) collecting a plurality of Doppler signals from muscle tissue of a heart, using a plurality of non-imaging transducers; and
(b) reconstructing a map of motion in said heart from said signals.

There is provided in accordance with an exemplary embodiment, a method of patient fluid tracking, comprising:

(a) providing a patient;
(b) attaching said patient to a fluid modifying system; and
(c) continuously tracking a fluid status of said patient by non-invasively monitoring at least one cardiac parameter.

There is provided in accordance with an exemplary embodiment, a method of cardiac parameter tracking in which an E' value of a patient is tracked on a beat-to-beat level in a continuous manner.

There is provided in accordance with an exemplary embodiment, a cardiac monitor, comprising:

(a) a wearable body;
(b) at least one sensor adapted to acquire Doppler signals form the heart; and
(c) circuitry adapted to extract at least one extreme event in a cardiac cycle from said signals.

## BRIEF DESCRIPTION OF THE DRAWINGS

In the following drawings, identical structures, elements or parts that appear in more than one drawing are generally labeled with the same numeral in all the drawing in which they appear. Dimensions of components and features shown in the drawings are chosen for convenience and clarity of presentation and are not necessarily shown to scale. The drawings are listed below.

Fig. 1 is an illustration of a prior art hemodynamic monitoring system operating using Swan Ganz catheters;
Figs. 2A-2G illustrate the usage of a prior art modem Echocardiography scanner;
Fig. 3 is a schematic diagram of a monitoring device, in accordance with an exemplary embodiment of the invention;
Figs. 4A-4B illustrate cardiac anatomical landmarks, for use in accordance with exemplary embodiments of the invention;
Figs. 5A-5C illustrate various configurations of ultrasound transducers and ECG electrodes on the anterior chest in accordance with exemplary embodiments of the invention;
Fig. 6 is a flowchart of a monitoring cycle, in accordance with an exemplary embodiment of the invention;

Figs. 7A and 7B illustrate probe relative locations and signals measured thereby in accordance with an exemplary embodiment of the invention;

Figs. 8A and 8B illustrate Doppler signals in certain situations, in accordance with exemplary embodiments of the invention;

Fig. 9 is a flowchart of a method of signal processing in accordance with an exemplary embodiment of the invention;

Fig. 10 is a flowchart of a method of structure velocity and location estimation, in accordance with an exemplary embodiment of the invention;

Fig. 11 is a schematic showing of angle independence viewing using a single apical transducer, in accordance with an exemplary embodiment of the invention; and

Figs. 12A and 12B illustrate results of measurements, in accordance with an exemplary embodiment of the invention.

## DETAILED DESCRIPTION OF EXEMPLARY EMBODIMENTS

### Overview

A cardiac monitor in accordance with some embodiments of the invention is a non-invasive system for continuously assessing heart pressures and myocardial function during contraction and relaxation. The system combines blood Doppler and tissue Doppler measurements and is optionally based on well validated measurements for assessing clinical parameters. The system acquires data from a distributed array of ultrasound transducers (3-6 transducers, but in some embodiments fewer or more, for example, 1 or 8) that are placed over the anterior chest wall in predetermined anatomical sites (optionally using a procedure similar to that of positioning ECG monitor leads now commonly used in intensive care units). The raw data of the received signals are analyzed in real-time in the computing unit to generate a 3 dimensional Doppler (both blood flow and tissue) map of the heart.

Processing derives from the Doppler data one or two or more of the early diastolic transmitral flow velocity (E), the late diastolic transmitral flow velocity as denoted by A, the late diastolic myocardial wall velocity as denoted by A', the myocardial wall velocity during early diastole (E') and systole (S'). The latter are direct measures of diastolic (for E') and systolic (S') myocardial function respectively; that is the relaxation function and contraction function of the heart muscle. The left ventricular diastolic pressure (LVDP) is assessed from the ratio E/E'. The velocity time integral (VTI) is optionally derived from analysis of the Doppler maps and the SV is calculated by multiplying by the measured left ventricular outflow tract (LVOT) area. The cardiac output is optionally estimated by multiplying SV by the heart rate (HR).

In an exemplary embodiment of the invention, the clinical parameters are displayed numerically and graphically on the system screen and are stored in the Storage Unit. Optionally, the monitor displays measurements continuously (or at any other rate as determined by the clinical staff), optionally, with no limitation on patient posture. Optionally, the system is used for any desired time length with little or no fear of infection or other problems typically caused by invasive devices.

A particular feature of some embodiments of the invention is that the system can be positioned on a patient and yield usable results without the intervention of an expert and/or without using an imaging step to position the system. In some embodiments, once the system is positioned, even using an expert and or imaging for guidance, no expert is need to maintain the system in a usable positioning, for appreciable periods of time.

A particular feature of some embodiments of the invention is that the data is acquired as scalar values, for example using a single transducer or transmitter-receiver pair. Optionally, signal analysis is used to determine a spatial area of interest, for example, based on the signal being at an extreme value (e.g., maximum or minimum).

A particular feature of some embodiments of the invention, is that the muscle signals and blood signals are acquired in a same cardiac cycle, possibly providing meaningful results even in arrhythmic patients or other patients with large beat-to-beat variability.

### Exemplary hardware configuration

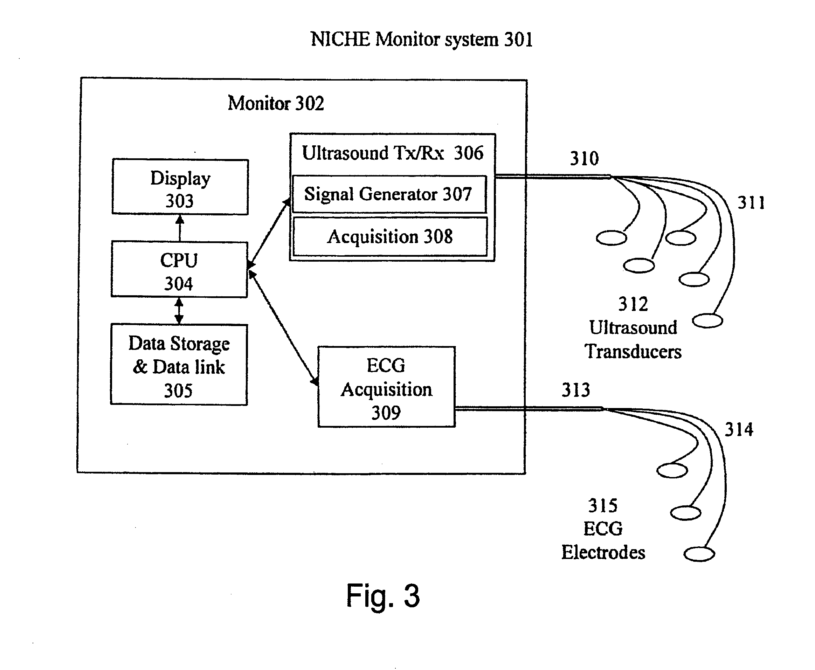

Fig. 3 illustrates an exemplary monitoring system 301 including a monitor unit 302, an ultrasound probe section 310, and an ECG probe section 313.

Optionally, a processing means such as a personal computer (e.g. Pentium-type computer with added hardware cards for signal generation and acquisition and optionally signal processing) serves as the monitor unit 302. Unit 302 includes a control and processing unit 304, data storage and optional data link unit 305, an optional display unit 303, an ultrasound transmitter/ receiver unit 306 that consists of an ultrasound signal generator 307 and acquisition unit 308, and an ECG acquisition unit 309. In some embodiments, the ECG and/or ultrasound sub- systems are separate, optionally external, modules which are controlled by CPU 304 and/or send data thereto. In a particular embodiment of the invention, the ultrasonic sub- system and the ECG are standard devices, such as an echo- cardiography device with an ECG detector built in and monitor unit 302 processes data received therefrom. For example, a single transducer embodiment, as described below, may be integrated into an imaging sensor and/or system. Optionally, ultrasonic signal processing and/or ECG processing is performed by such separate units.

In some embodiments of the invention at least some ultrasonic processing is carried out by unit 302, for example one or more of determination of the relative locations of chest transducers, Doppler signal extraction, processing generation of spatial map (if any) with attribution of Doppler signals, measurement of S', E', and/or E, and/or calculation of the ratio E/E'.

In an exemplary embodiment an ultrasonic application section 310 is provided with one or more transducers, for example, 3-6 ultrasound transducers 312 that are connected to ultrasound Tx/Rx unit 306 by one or more leads 311. Optionally, the leads comprises cables for transmission and/or reception and/or control lines. The transmission and reception cables are optionally made of either double-shielded coaxial cable or twisted pairs or any other type of wiring suitable for ultrasound operational frequencies.

In an exemplary embodiment of the invention, the transducers comprise separate transmitters and receivers (e.g., one or more of each). Optionally, at least some of the transducers act as both receivers and transmitters. Optionally, the transducers are simple transducers which provide a single beam (optionally using a shaping or a lens to shape the beam). The transducers are optionally single element transducers. Optionally, one or more of the transducers is an array transducer, for example, a phased array (such as annular or linear) element.

Optionally, the ultrasound transducers are formed of PZT, cMUT or any other suitable material for Doppler ultrasound transmission and/or reception. In an exemplary embodiment of the invention, the contact surface between the transducer's face and the skin is less than 1 cm in maximum dimension (e.g., 1 cm$^2$) , so that the transducer fits in the interspaces between the ribs. One or more of the transducers may exhibit a wide beam profile, for example as described below. Optionally, broad beams are provided using spherically shaped transducers or by using designated lenses to diverge the ultrasonic beam. Optionally, the beam is not circular in cross- section, for example, being elliptical with a height- width ration of between 1: 1.2 and 1: 6, or intermediate values, such as 1: 2 or 1: 4.5) . Optionally, the beam is steered, for example, by rotation, mechanically and/or electronically.

In an exemplary embodiment of the invention, the ultrasound transducers and/or leads are reusable and mechanically durable. Optionally, the transducers are provided with a disposable covering, for example, a means for attachment to the chest. Optionally, the means provides good acoustic contact, for example, including gel or a matching layer and is sized to match inter-rib spaces. Optionally, the means includes a lockable joint or a plastically deformable attachment, for example a chunk of putty, to allow controlling and fixing transducer orientation and/or distance between transducers. Optionally, the means includes a wireless transmitter. Optionally, the means includes ECG sensors and/or places therefore. Optionally, the means is a vest or a halter.

In an exemplary embodiment of the invention, one or more ECG leads 314 connect ECG acquisition electronics 309 to one or more ECG electrodes 315. In an exemplary embodiment of the invention, ECG signals are used to help arrange data in a temporal manner. In one example, QRS complexes are used to time electrical systole, thus allowing for proper identification of subsequent Doppler derived signals from mechanical systole (S', LVOT flow velocity envelope, VTI, SV) and diastole (E, E', A, A'). This analysis may be useful in analyzing data from subjects with frequent extrasystoles (premature ventricular beats and premature atrial beats), and irregular heart rhythms (e.g., as caused by atrial fibrillation). In an exemplary embodiment of the invention, monitor 301 is used to detect an actual effect of arrhythmia. Optionally, an ECG trace and parameters as described herein are shown together, for example, in an ECG trace. Optionally, one or more of the parameters as described herein are fed in a continuous manner into an ECG machine or a stress test monitor.

Depending on the application, ECG signals may be used merely for detecting the heart rhythm, in which case fewer leads may be sufficient, or for detecting activation pathologies which may impact when various peak velocities are expected. Optionally, a table or other conversion element is provided in system 301 to provide expected relative times of peak velocities for various pathologies.

Exemplary probe positioning

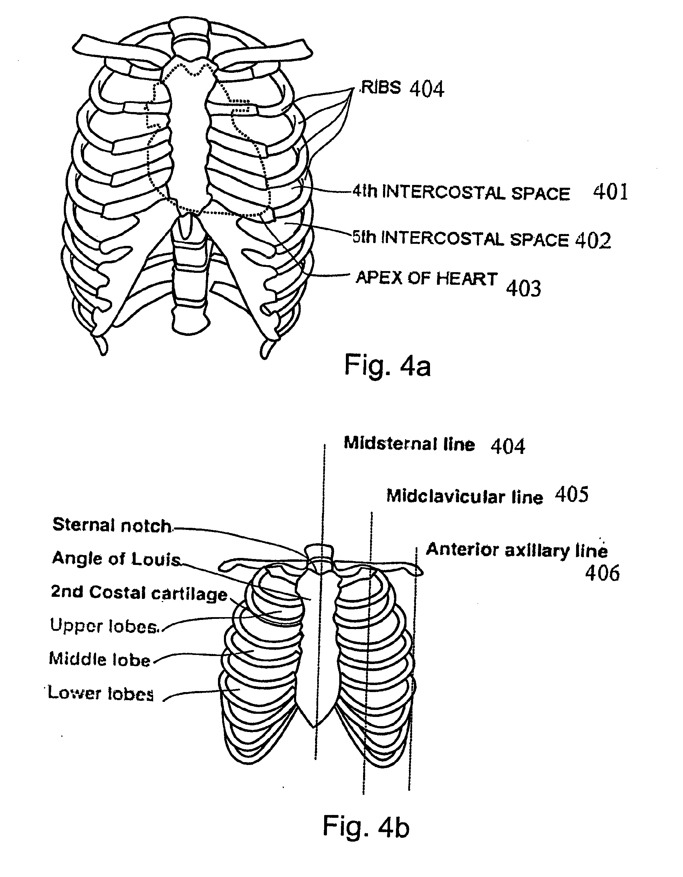

Figs. 4A and 4B show anatomical landmarks that are easily recognized by any medical staff member. These include ribs 404 and a 4$^{th}$ intercostal space 401 and a 5$^{th}$ intercostal space 402. An apex of the heart 403 is usually best accessed in the 4th or 5th intercostal spaces between a midclavicular line 405 and an anterior auxiliary line 406.

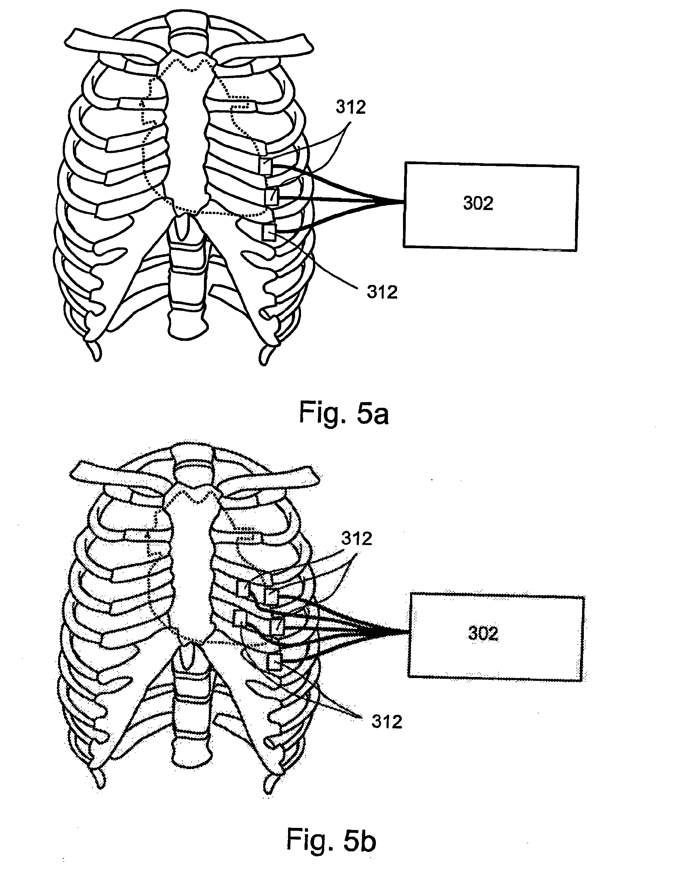

In a preferred embodiment of the present invention ultrasound data is gathered using a set of ultrasound transducers 312 that are spread on the anterior chest and arranged according to the anatomical landmarks discussed above. Fig. 5A illustrates an embodiment in which transducers 312 are placed in the 5th, 4th and 3rd intercostal spaces between the midclavicular line and the anterior axillary line. In another exemplary embodiment, additional transducers are placed in the interspaces between the ribs in convenient acoustic paths. Fig. 5B shows an example of an embodiment for 5 transducers 312 where the additional two transducers are placed in the 3$^{rd}$ and 4$^{th}$ intercostals spaces and between the midstemal line 404 and the midclavicular line.

In an exemplary embodiment of the invention the ultrasonic data is acquired using one or more transducers that are non-focused. In this embodiment the beam profile of one or more transducers is relatively wide, in order to cover larger volume. For example the transducer beam profile may have -6dB attenuation angle of $\pm 30$ degrees, or $\pm 20$, $\pm 45$ or $\pm 60$ degrees or intermediate or greater values .

In an exemplary embodiment of the invention, the transducers transmit and receive pulses at an operating frequency of 1-4 MHz, and a pulse repetition rate (PRF) up to 6 kHz controlled by software, and a burst (number of cycles in a pulse) of between 2-16. In an exemplary embodiment of the invention, the ultrasonic probes are attached to the skin using adhesive tape (e.g., similar to ECG) or alternatively with straps. It should be noted that in some embodiments of the invention, the duration of attachment to patient is long - up to few days or weeks.

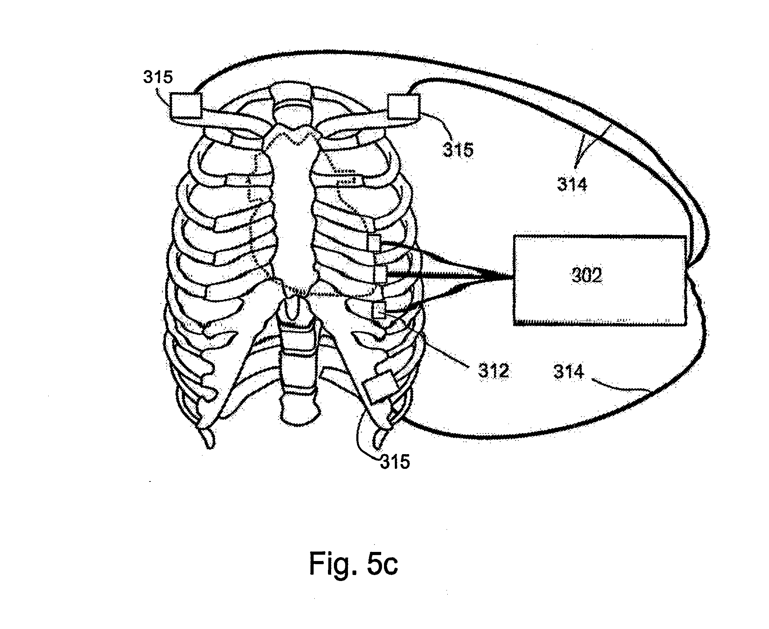

In an exemplary embodiment of the invention (e.g., Fig. 5C), a continuous trace of echocardiogram and ultrasonic signal is provided. Optionally, electrocardiographic electrodes 315 are placed on the right shoulder, left shoulder and left leg or lower abdomen. Optionally, the ECG input is used to establish onset of electrical systole. In an exemplary embodiment of the invention, onset of electrical systole is used to determine sampling time in patients with complex cardiac activation, for example, patients with frequent extrasystoles or patients in atrial fibrillation.

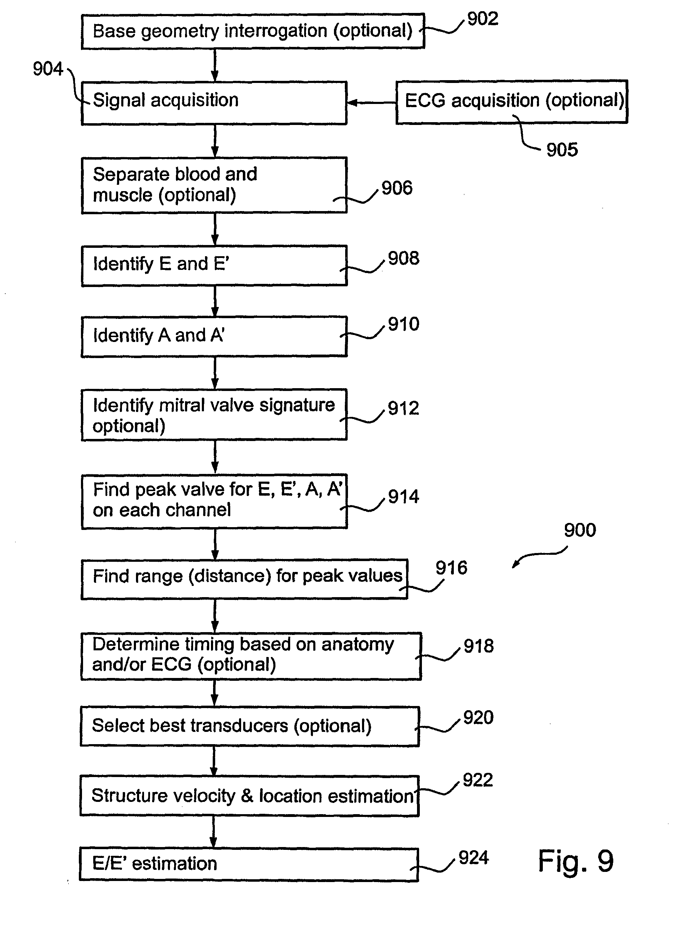

In a preferred embodiment of the present invention three or more ultrasound transducers 312 are used to acquire Doppler signals which can be used to reconstructed 3D motion vectors of muscle and blood. In an exemplary embodiment of the invention, the acquisition uses a multigate of 16, or 32 or 64 range gates starting from 40 mm (or 20 or 60) to 120 or 150 mm, controlled by software and generally depending on the expected or known anatomy of the patient. In an exemplary embodiment of the invention, the data acquisition comprises an acquisition sequence in which one or more transducers transmit while the other transducers receive. An exemplary method of reconstructing a 3D vector is described later below with respect to Fig. 9.

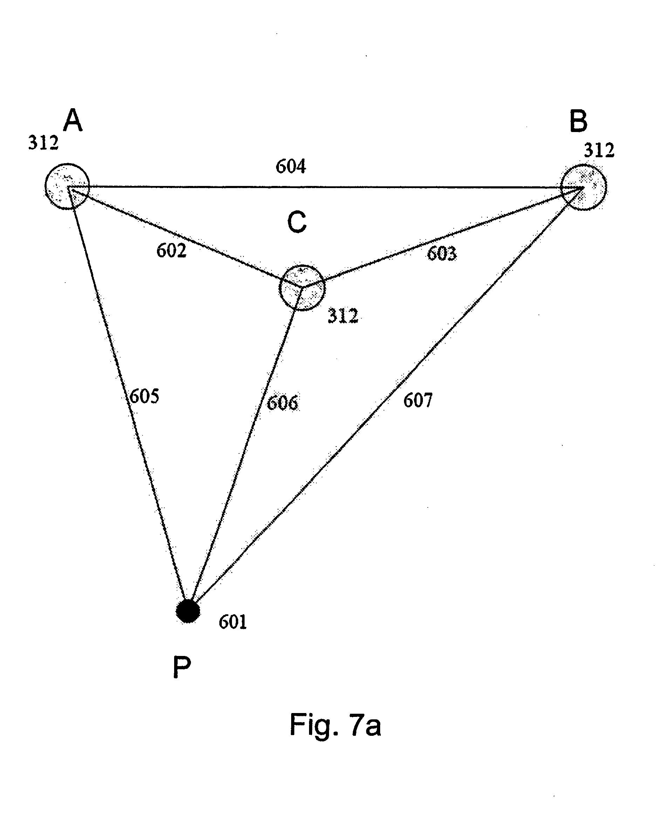

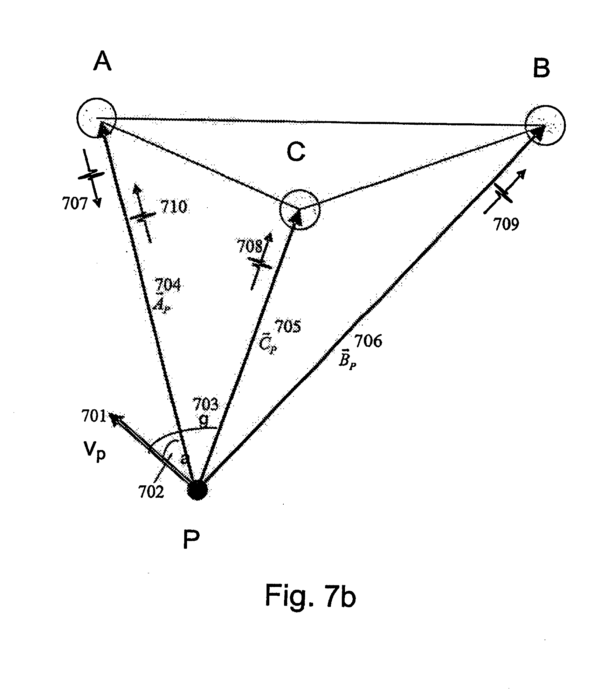

In an exemplary embodiment of the invention the relative distances between the transducers 312 are calculated using a "time of flight" method derived from their ultrasonic signals. An exemplary method is described below with respect to Figs. 7A and 7B. In another embodiment the distances are measured by other means such as but not limited to air ultrasound, optical, RF, magnetic or mechanical means. In another embodiment the distances are fixed or settable and then measured using a setting element (e.g., a digital ruler).

<u>Exemplary operation</u>

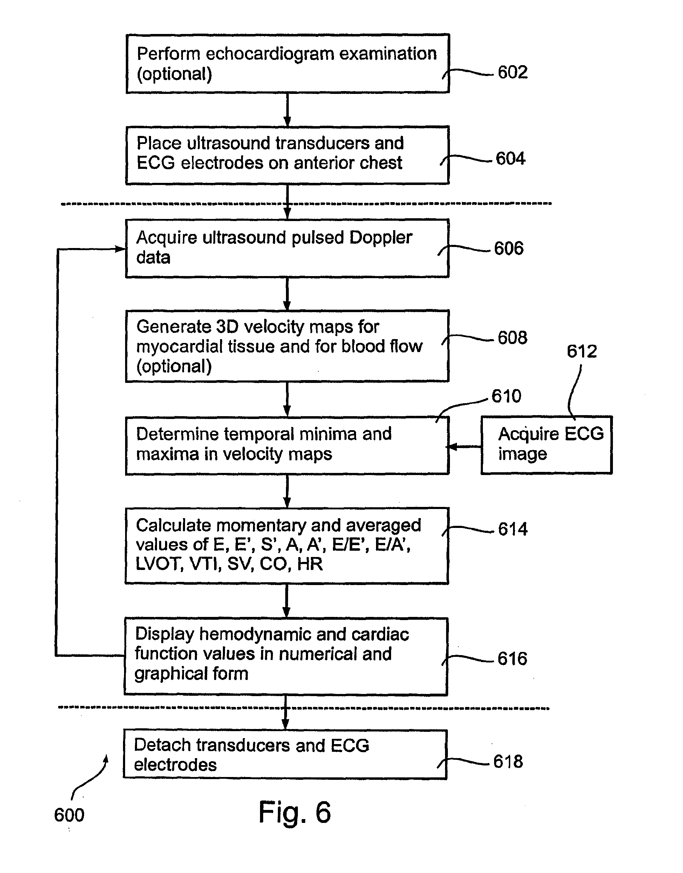

Fig. 6 is a flowchart 600 of an exemplary method of use of system 301.

At 602, an echocardiographic examination is optionally performed, for example, to the general layout and activity of the heart and/or to check for pathologies that might interfere with the sensing. In some embodiments of the invention, imaging of the heart is used to guide the placement of probes.

In an embodiment of the present invention, an echocardiogram scan is optionally performed prior the initialization of the monitoring. Optionally, the scan is used to indicate the site for best apical views. In an exemplary embodiment of the invention, monitor 302 (e.g., a planning module thereof) uses this input to determine a best configuration for ultrasonic transducers and to guide the user where to place the transducers. In an exemplary embodiment of the invention, the imaging probe is aimed at various spots on the chest for locating good ultrasonic windows (e.g., according to signal strength, tissue velocity, and/or range values). Optionally, the echo input used to align the transducers in a correct direction.

Optionally, the scan is used to perform baseline measurements of E, E', E/E', S', A, A', LVOT flow velocity envelope, VTI and/or SV;

Optionally, the scan is used to measure distances in the heart or between the transducers and the heart, for example, to measure the distances from transducers' attachment sites to apex of the heart, to mitral annulus and/or to the aortic valve during systole and diastole. These distances are expected to vary between patients depending on their gender, weight, height and other factors. In an exemplary embodiment of the invention, the measured distances are used by monitor 301 in embodiments where an extreme value is searched for within a volume and/or to reduce possible artifacts due to undesired pickup of signals from other regions. Optionally, a user indicates on such an image what volume to look at. Optionally, distances obtained by imaging are used to set a multigating range

Optionally, imaging is used for geometrical measurements in the heart, for example for LVOT area determination, LA area or volume measurements, and/or for detection of pathologies such as aortic stenosis, aortic insufficiency or Mitral stenosis. In some embodiments, various pathologies are detected using non- imaging means. For example, aortic insufficiency is determined using non- imaging means, based on a correlation of the insufficiency with a deceleration of an aortic regurgitation jet (detectable using methods described herein) . Typically, the latter implies that velocities exceeding 1.7 m/s may exist and may be undetectable due to aliasing. In some cases, the pathology is used to change the operating mode, e.g., to a higher PRF. Optionally, the measurement of LVOT area is entered into monitor 302 to help in deriving SV. In case of aortic stenosis the aortic valve area is optionally entered instead. With aortic insufficiency the direction of the aortic regurgitant jet is optionally noted and considered (e.g., detected in the signal and not counted) in a subsequent optional 3- D rendering of diastolic Doppler velocities. In an exemplary embodiment of the invention, aortic regurgitation is detected using a non- apical transducer transmitting at an oblique angle of incidence. In the event of mitral stenosis the user may consider not using the methods described here, as the cardiac loading parameters may be affected. Optionally, monitor 301 is integrated with or connected by a data connection to the imager, so that indications indicated on the image or measured by the echo system, can be passed and used by monitor 301.

At 604, ultrasonic and ECG sensors are placed on the patient, optionally permanently attached. In some cases, the attachment is made final when meaningful measurements are made.

At 606, Doppler data from muscle and blood is acquired. In some embodiments, only blood or only muscle data are used. One example of using only tissue data is when more than 3 transducers are available and a crude 3D velocity map of tissue is generated to view the general structure of the heart or a portion thereof. It may be simpler to use tissue rather than blood data as tissue generally has a smaller signal and since velocities are lower, it is easier to provide a sequence where each transducer transmits in turn, with the same PRF and without aliasing. Optionally, such a map has between 10 and 64 voxels.

At 608, 3D velocity maps are generated for muscle and blood. In some embodiments, the maps generated are 1D (e.g., along a transducer beam), however, the map may include a 2D or a 3D vector. In some embodiments, measurements of only a small number of points (e.g., 1-6 voxels) is provided, rather than maps. In some embodiments, vectors are calculated which represent a behavior of muscle and/or blood, but which do not necessarily correctly reflect any particular point in space.

In an exemplary embodiment of the invention a reconstruction module constructs two separate 3D velocity maps from Doppler data: one map for muscle and one map for blood flow.

At 610, maxima and/or minima are identified in the maps. In some embodiments, only maxima and/or minima measurements are extracted from the ultrasound data, obviating need for map generation. In an exemplary embodiment of the invention, the maxima/minima are temporal maxima/minima. Optionally or alternatively, the extreme values identified are local spatial extremes. Optionally, the identified values are not the most extreme values but near extreme values (e.g., within 20% or 10% of extreme), for example, to account for noise or to account for an effect of smoothing or other filtering.

Optionally, after the maps are generated a search is made (optionally within range parameters that depend on the heart size, shape and/or location) , for a temporal peak (regional or global) . Using the ECG input monitor 301 optionally associates the timing of the obtained values with hemodynamic parameters.

At 612, various cardiac parameters may be extracted from the measured values, for example one or more of E, E', S' A, A', E/E'. E/A, LVOT, VTI, SV, CO and/or HR. The extracted values may be presented in raw form, averaged and/or otherwise filtered, for example, by smoothing, or by binning to match different cardiac rhythm morphologies.

In an exemplary embodiment of the invention, the diastolic transmitral flow velocity E is derived from blood flow three-dimensional velocity map, by finding the spatial maximal during diastole. Pick up of undesired signals from the tricuspid valve inflow and RV free wall motion is optionally eliminated by analyzing the spatial information and tracing the origin of these signals. Optionally, such elimination uses the detection of local maxim and distinguishing between left and right sides. In an exemplary embodiment of the invention, the late transmitral flow velocity A is derived from the three-dimensional blood flow velocity map, by finding the second velocity peak following E and corresponding to the P wave on a simultaneous ECG tracing during diastole. Optionally, in the absence of P waves monitor 301 will not search for A waves.

In an exemplary embodiment of the invention, the myocardial wall velocities E', A' and S' are derived from the three-dimensional myocardial velocity map and calculated as the maximal tissue velocities pointing away from (E' and A') and towards (S') the apex of the heart during diastole and systole, respectively. The maximum tissue velocity is associated with the myocardial tissue at the level of the mitral annulus. In an exemplary embodiment of the invention, the association is by using a geometrical reference such as the mitral valve leaflet or the calculated origin of peak transmitral blood flow velocity, and from there estimate the level (distance) of mitral annulus and restrict processing to signals from that area.

In general the velocity vector comprise of three components: longitudinal, radial and circumferential. An apical transducer (e.g., even if only one is used) will pick up primarily the diastolic and systolic components of longitudinal muscle function to give E' and S'. The late diastolic myocardial wall velocity A' is optionally derived from the three-dimensional myocardial velocity map, by finding the second tissue velocity peak pointing away from the apex of the heart (optionally using only an apical transducer and not a 3D map) and corresponding to the P wave on the simultaneous ECG tracing during diastole. Since E and E' as well as A and A' are conjugates and are apparent nearly at the same time, generally identifying one allows the other to be understood to exist and thereby be detected. Optionally, a strong bipolar Doppler signal associated with mitral valve leaflet opening and closing is identified. The calculated origin of this signal is optionally used as a "good" spatial reference point for the measurement of E, A, E', A' S'. For example, this signature can be used for defining a volume of interest and to differentiate lateral from septal side of the signal. In an exemplary embodiment of the invention, this is done by identifying the unique leaflet signal in space and comparing its position to that of the peak velocity. Optionally, the timing of the signal is used to identify the onset and the end of diastole

In an exemplary embodiment of the invention, the LVOT velocity profile is derived from the three-dimensional velocity map during systole. A global maximum velocity is sometimes not a good parameter since mitral regurgitation that (almost always is present and) also occurs in systole usually has a much higher velocity than LVOT velocity. Optionally, timing is used to distinguish in a broad-beam system. In mitral regurgitation the systolic flow starts earlier than the LVOT flow. The LVOT flow usually starts after the "isovolumetric contraction period" which can be seen on the tissue Doppler signal, and is about the same time as the onset of the S' wave. Optionally, the LVOT velocity is singled out from the velocity map by either looking at a spatial maximum away from the mitral valve, or by tracking the timing of onset of the flow, or a combination of both methods.

In an exemplary embodiment of the invention, the LVOT area is directly calculated from 3-dimensional Doppler data. Typically, the interface between simultaneous 3-dimensional tissue Doppler and 3-dimensional blood flow Doppler maps through the LVOT corresponds to the endocardial border of the LVOT. During systole (e.g., in the absence of significant aortic stenosis) the narrowest part of the LVOT is at the aortic valve annulus. Since the LVOT is an essentially circular tube at this point the area can be calculated by the formula: $A = \pi(D/2)^2$ where D is the diameter at the annulus. D is optionally determined form the map or based on a preliminary imaging.

In another embodiment, entry of calculated LVOT area, by a user or from another source, is supported. Generally, there is no significant variability of the LVOT diameter in a patient, so an initial LVOT area can be derived for example from a routine trans-thoracic echo and then set for a monitoring process. This methodology may also be useful in patients who have aortic stenosis previously determined by echocardiography. In these patients the effective aortic valve area (usually calculated by continuity equation) is optionally entered instead of LVOT diameter and should give the SV when multiplied by the VTI.

In another embodiment, a default LVOT area of 3.1415 cm$^2$ (i.e. $\pi(1)^2$) is used if no LVOT area is entered. This value corresponds to a LVOT diameter of 2 cm, which serves as a good estimate for the majority of patients.

It is a particular feature of some embodiments of the invention that tissue Doppler and blood flow Doppler signals are collected during a same cardiac cycle. In an exemplary embodiment of the invention, the tissue velocity vector map and the blood flow velocity vector map are measured simultaneously during a same cardiac cycle.

In an exemplary embodiment of the invention, a momentary measurement of an hemodynamic parameter that combines tissue velocity vector, blood flow velocity vector or both, such as but not limited to the ratio of E/E', is carried out in a single cardiac cycle. This can be particularly advantageous in patients with intra-beat variations, such as patients with frequent extrasystoles (early beats) or irregular rhythms such as atrial fibrillation.

At 614, the extracted values are optionally displayed in numerical and/or graphical form. Optionally, raw data (e.g., Doppler signal trace) is shown as well. Optionally, the data is overlaid on an image of the beating heart, if acquired. Optionally, other modalities than ultrasound are used for acquiring the image, for example, MRI, X-Ray CT and/or nuclear medicine imaging. Optionally, the imaging is carried out at a same time as ultrasonic monitoring, for example, using a probe that is not affected by the imaging modality (e.g., radiation transparent and non-scattering for x-ray and gamma radiation, and non-magnetic for MRI).

The monitor displays measurements continuously (or at any other rate as determined by the clinical staff), optionally with no limitation on patient posture. In some embodiments, an accelerometer or other sensor is used to detect posture change. Optionally, when a posture change is detected, the system is recalibrated, for example, by performing base triangulation and/or by selecting again a set of transducers with a best signal. In an exemplary embodiment of the invention, such a selection step is carried out only periodically and it is assumed that the previously chosen transducers are suitable for a period of time, unless the signal goes bad or another indication (e.g., accelerometer) indicates posture change occurred.

In an exemplary embodiment of the invention, averaged measurement of hemodynamic parameters is performed by averaging momentary hemodynamic measurements over two or more cardiac cycles.

In an exemplary embodiment of the invention, E is obtained for each beat and averaged over five or more cardiac cycles. The averaged value is displayed to determine the mitral valve inflow velocity on a continuous basis. This value reflects the left atrial pressures, or the "preload"

In an exemplary embodiment of the invention, E' is obtained for each beat and averaged over five or more cardiac cycles. The averaged value is displayed to determine diastolic myocardial function on a continuous basis.

In an exemplary embodiment of the invention, E/E' is obtained for each beat and averaged over five or more cardiac cycles. The averaged value is displayed to estimate left ventricular filling pressure on a continuous basis.

In an exemplary embodiment of the invention, S' is obtained for each beat and averaged over five or more cardiac cycles. The averaged value is displayed to determine systolic myocardial function on a continuous basis.

In an exemplary embodiment of the invention, maximal LVOT Doppler velocities and velocity time integrals are obtained for each beat and averaged over five or more cardiac cycles. The stroke volume and cardiac output are calculated and displayed on a continuous basis.

Optionally, acts 606- 616 are repeated continuously for as long as desired for example, hours, days or weeks.

Optionally, the location of the probe is marked on the body using a marker, so that probes can be returned after cleaning of the skin, for example.

At 618, transducers and/or ECG sensors are optionally disconnected. Optionally, the transducers are reusable and are optionally sterilized before reuse.

In a particular implementation, the following methodology is followed:

(a) Acquire tissue Doppler signals of the myocardium at the level of the mitral annulus. Typically this has three distinct components: S', E' and A' waves. Peak S' and E' amplitudes are optionally measured separately for every beat and continuously averaged (e.g., with the four or more previous beats).

(b) Acquire blood flow Doppler signals of mitral valve inflow. This flow typically has two components: E and A waves. Peak E amplitude is optionally measured for every beat and continuously averaged (e.g., with four or more previous beats). A is optionally not used.

(c) Calculate the ratio of the E wave amplitude divided by the E' amplitude. The ratio E/E' is measured separately for every beat and continuously averaged (with the four or more previous beats).

(d) Optionally measure the left ventricular outflow velocities and derive the velocity time index (VTI). VTI is optionally calculated with every beat and averaged with, for example, the previous four or more beats. A default LVOT area of 2 cm$^2$ is used unless user enters a different (e.g., echo derived) value;

(e) Display the averaged values for E, E', E/E', S' and SV and CO, in numerical and/or graphical forms.

(f) Store the momentarily and averaged values in a local archive and optionally transfer the data to a remote station for further displaying, processing and/or storage.

Exemplary applications

The above described monitoring system in various embodiments thereof, may be used for a range of applications, including one or more of hospital applications, non-hospital applications, screening, diagnostics, monitoring and/or as a control element.

According to the invention, monitoring is for a period of time of at least 10 minutes for example, 30 minutes, 1-3 hours, 10 hours, 1-3 days, a week or longer periods. Optionally, during the monitoring measurement is made of every beat except for beats where the signal is bad or during calibration. Optionally, the displayed results are filtered, for example, a result being the average or other function of measurements during several (e.g., 1, 3, 5, 10, or intermediate or more beats). Optionally, not every beat is measured, for example, only one in every 2, 5, 10, 30 or other number of beats is measured. Optionally, only a best measurement (e.g., with respect to signal quality) of every N beats is used. N may be, for example, 2, 4, 10, 20, 60 or intermediate or greater numbers.

In an exemplary embodiment of the invention, monitoring comprises providing a live signal optionally with one or more alerts set (e.g., minimum, maximum, variability, signal quality) for one or more parameters or functions of parameters. Optionally, a storage of last 24 hours and/or of acute events is provided.

In some embodiments of the invention, the monitoring is of absolute values. In some embodiments, the monitoring is of changes. While only some parameters are explicitly described below, monitoring and/or diagnosis may use any of the cardiac parameters known in the art, including as described in the background.

Optionally, monitoring is not continuous. For example, an external trigger, such as a blood pressure being taken or a change in blood pressure detected (or other physiological measurement) is used to trigger measurement. Optionally or alternatively, the measurement is based on a time schedule, for example, once a minute, once in 10 minutes or once an hour.

In a hospital application, system 301 is optionally connected with various hospital systems, such as patient information systems, imaging systems, diagnostic result systems and bedside monitoring systems. In such a connection, data may be read into the system and shown with (or used for extracting) the measured parameters and/or measurements may be sent to an external system. Optionally, the integration is with ICU (intensive care unit) systems, for use of system 301 in an ICU.

In an exemplary embodiment of the invention, in a hospital situation, system 301 is reusable and the probes sterilizable. Optionally, the parts in contact with the body are disposable, for example, ECG electrode pads and/or transducer coverings.

In exemplary monitoring applications, monitoring system 301 may be connected to a bed side monitor and data provided therefrom sent to a nurse station. Optionally, alert settings may be provided to indicate problematic (expected) cardiac conditions. In an exemplary embodiment of the invention, the system has a potential advantage that it may be used for a long period of time without side effects.

In exemplary home applications, broad beam transducer(s) are used to simplify self testing or usage by a low-qualification caregiver. Optionally, stable measurements allow comparing a baseline value from more than a few minutes (e.g., hours, days or weeks) to a current value to detect changes and/or track changes.

In exemplary diagnostic applications, the system is readily available and requires no advance preparation of a patient.

In exemplary screening applications, the duration of a test is short, with no preparation, side effects or required after-treatment.

In exemplary ambulatory applications, use is optionally made of the broad-beam nature of some embodiments which allows substantial movement of transducers relative to body parts to occur without preventing useful signal acquisition. In an exemplary embodiment of the invention, operation time may be as follows. Transducer placement/installation and/or optimization assisted by software could take 1-5 minutes. The removal of transducer could take a few seconds. A minimum duration for a single meaningful and reliable measurement could take, for example, 10 seconds (including averaging over up to 10 heartbeats). To establish a baseline for longterm behavior a series of measurements over a period of 5 minutes or 10 minutes or 30 minutes or 1 hour or days may be used. For detecting clinically significant changes a series of 3-5 single measurements may be used (e.g., a duration of 30-60 seconds).

In a first exemplary application of system 301, postoperative patients are provided with hemodynamic monitoring, for example, to assess, detect and/or assist in treating side effects of surgery or of anesthesia provided during surgery. Optionally, a purpose of such monitoring is maintaining a E/E' ratio under 15 (which correlates to a LVDP of <15 mmHg) or other levels that are determined clinically. Other threshold values may be provided. Optionally or alternatively, changes in E/E' are tracked, especially in response to therapy. Optionally or alternatively, SV is maintained at > 55 cc/min or other levels that are determined clinically. Optionally or alternatively, changes in SV and/or CO are tracked, especially in response to therapy. Optionally or alternatively, S' is monitored for changes in ejection fraction.

In another exemplary embodiment, patients in shock (or in which shock is a danger) are monitored, considering that various cardiac parameters are affected by the circulation problems associated with shock, so such parameters can indicate progress of the shock and/or provide warning signals. Optionally, system 301 is used to differentiate cardiogenic etiology (low SV and CO) from non-cardiogenic, i.e. septic shock (high SV and CO). Optionally or alternatively, during treatment, system 301 is used to maintain an E/E' ratio under 15 (which correlates to a LVDP of <15 mmHg) or other levels that are determined clinically. Optionally or alternatively, changes in E/E', especially in response to therapy, are tracked. Optionally or alternatively, SV is maintained at > 50 cc/min or other levels that are determined clinically. Optionally or alternatively, changes in SV or CO are tracked, especially in response to therapy. Optionally or alternatively, S' is monitored for changes in ejection fraction.

In another example, patients with myocardial infarctions are monitored, for example, to detect changes in cardiac parameters indicative of damage to heart and/or to assist in diagnosing an infract.

In an exemplary embodiment of the invention, an E/E' ratio under 15 (which correlates to a LVDP of <15 mmHg) or other levels that are determined clinically, are maintained or a change is looked for. Optionally, such maintaining (and/or in other embodiments) is by providing and/or changing treatments by medical personally or by automatic treatment systems, for example, a dosage control system. Optionally or alternatively, changes in E/E' are tracked, especially in response to therapy. Optionally or alternatively, SV is maintained at > 50 cc/min or other levels that are determined clinically. Optionally or alternatively, changes in SV or CO, especially in response to therapy, are tracked. Optionally or alternatively, S' is monitored for change in ejection fraction.

In an exemplary embodiment of the invention, patients, for example, with coronary arterial disease or in suspected infract patients are monitored to detected reduction in left ventricle systolic function (or other function) that is indicative of ischemia. For example, a reduction in E' or E/E' may be followed continuously, displayed and/or stored. Optionally, such reduction is used to reinforce an indication which is detected in an ECG signal. In a patient with heart rate variability or arrhythmia, detection of a change may be limited to "normal" beats, as indicated by ECG or runs of such beats. In some cases, the results are binned by beat type and a change I any of the bins may be tracked. IPA a reduction in E' form a baseline, of, for example, 10%, 20%, 30% or intermediate or greater values may be of interest. Optionally or alternatively, a reduction form "normal" values of >10 to abnormal values of <8 may be tracked.

In an exemplary embodiment of the invention, such tracking is used to diagnose ischemia (or determine if pain is caused by ischemia), if the affected area is large enough to affect cardiac function.

In an exemplary embodiment of the invention, hemodynamic monitoring of patients undergoing hemodialysis, is provided. Depending on the results of such monitoring, the rate of treatment may be changed and/or averting therapy may be applied. Optionally or alternatively, changes in E/E' are tracked especially in response to therapy. In an exemplary embodiment of the invention, a predetermined goal can be established as a hemodynamic equivalent to "dry weight". Optionally, this goal is a baseline from the patient or a clinically decided value. Optionally, if the values do not change (at a desired rate and/or direction) in spite of expected change due to hemodyalisis activity, an alert is generated. Such an alert can be instead of or additionally to an alert generated by problematic cardiac parameter values. Optionally or alternatively, SV is monitored in response to therapy.

In an exemplary embodiment of the invention, hemodynamic monitoring of ambulatory surgery patients is provided. Optionally, such monitoring uses a property of some embodiments of the invention that the system is robust under movement.

In an exemplary embodiment of the invention, hemodynamic monitoring of burn patients, trauma patients and/or other difficult patients, such as children, mentally handicapped and neonates, is facilitated, for example because arterial access (which may be especially problematic with such patients) is avoided. In an exemplary embodiments of the invention, diagnosis of such patients is facilitated because they do not necessarily need to be moved to have their cardiac functions assessed.

In an exemplary embodiment of the invention, patients are diagnosed, monitored and/or monitoring started outside the hospital, for example, by EMS services, for example, for trauma patients or patients with suspected cardiac and/or vascular problems.

In an exemplary embodiment of the invention, system 301 is used for monitoring a patient during surgery, for example, to track and/or detect an effect of the surgery and/or anesthesia.

In an exemplary embodiment of the invention, system 301 is provided as part of a medical provision system which, for example, selectively provides fluids or diurics to control a fluid balance of a patient.

In an exemplary embodiment of the invention, system 301 is incorporated with or associated with an external pacemaker (electrodes may be external or internal), to provide warning of hemodynamic problems and/or to provide a demand-pacemaker function.

In an exemplary embodiment of the invention, a device as described herein is used for tracking and/or diagnosing cardiac/hemodynamic problems in pregnant woman, for example, Preeclampsia, Eclampsia and/or maternal heart disease. Optionally, E/E' is tracked.

In an exemplary embodiment of the invention, system 301 is used as part of a stress test. Optionally, system 301 provides added feedback as to the progress of the test and/or cardiac health. Optionally or alternatively, cardiac output or other cardiac parameters are used as the target that is monitored by the test, instead of or in addition to heart rate. In an exemplary embodiment of the invention, E' and/or E/E' are used as indicators of ischemia. In an exemplary embodiment of the invention, during a stress test, various cardiac parameters are manipulated, for example, pre-load, to see the combined effect of modifying parameters and stress. Stress-free diagnosis may also be provided in such a manner.

In an exemplary embodiment of the invention, system 301 is used for screening, for example, being loaded into a vehicle and/or placed in public locations.

In an exemplary embodiment of the invention, system 301 is packaged as a worn Holter-like halter-type device, with batteries (e.g., rechargeable) and optional in-circuit signal processing to extract cardiac parameters of interest. Various body attachment mechanisms can be used in such a device, for example, a belt, straps, an undershirt or adhesive. Optionally, such extracted parameters are stored on a storage component of the halter-like device. Optionally or alternatively, raw data is stored. Optionally, the data is partially pre-processed before storing, for example, compression and/or calibration against a known baseline acquired using an imaging scanner. Optionally, the device includes measurement of other parameters, such as acceleration, breathing, ECG, EEG and/or blood pressure. Optionally, the device is used for generating an alert to a patient, rather than merely for data collection and includes, for example, a speaker or other output device. Optionally, the device includes a communication system, for example, a cellular RF circuit, for sending data and/or receiving instructions remotely.

In an alternative form factor, the ECG sensors and ultrasonic probes are worn, and part of the device (e.g., processing) is separate, connected by wired or by a short-range wireless link. Optionally, the sensors and probes are provided in a vest or patch, such as a type and/or design known for ECG measurement.

In an alternative form factor, a stand-alone hand-held probe is provided, optionally, with no power cord. In an exemplary embodiment of the invention, the tip of the probe comprises a single broad-beam ultrasonic transducer and also includes an ECG sensor, if needed (e.g., in some embodiments the cardiac cycle is deduced from the Doppler signal morphology). Optionally, the probe includes signal processing circuitry, a power supply (optionally a rechargeable battery) and a display. Optionally, a special indicator is provided to indicate if the measured signal is meaningful or not, to aid in aiming the probe.

In an exemplary embodiment of the invention, system 301 is used to detect and store (optionally in an on-board memory) a base-line value for one or more cardiac parameters. Thereafter, for example to detect an acute condition, a new measurement is made and compared to the base line. Optionally, the detection is made in a home setting. Optionally, the storage of the baseline value is at a remote monitoring station. Optionally, system 301 includes a connector to a telecommunication device (e.g., cellular telephone) or data line. In an exemplary embodiment of the invention, system 301 is used to detect indication of onset, improvement and/or adverse events in pulmonary hypertension, ischemia and/or congestive heart failure.

In an exemplary embodiment of the invention, a single apical sensor is used as a sensor for an implanted pacemaker, for example for assessing cardiac functionality for a rate-limited or demand pacemaker. In an exemplary embodiment of the invention, the sensor is implanted inside the body, for example, in the abdominal area and pointed towards the heart apex and its broad beam allows it to function even when the body distorts or if the sensor drifts somewhat in the body. Optionally, A-wave duration is measured as an indication of a need to modify A-V delay.

In an exemplary embodiment of the invention, system 301 is used during catheter procedures, as it allows various parameters to be assessed in a hands-free manner and without another person near the patient holding a probe and/or to maintain sterility.

In an exemplary embodiment of the invention, system 301 is coupled to other sensors which measure physiological parameters away from the heart. Optionally, system 301 correlates cardiac parameters with such other parameters on a beat-by-beat level. This may be useful, for example, to assess a connection between cardiac arrhythmia and syncope. In another example, the sensed measurements are used to detect problems with the operation of a cardiac assist device, such as an intra-aortic balloon (which tends to increase LV pressure). Optionally, the sensing is used to fine-tune the operation of such an assist device, for example, to optimize output and/or minimize problems.

In an exemplary embodiment of the invention, monitor 301 is used, for example, in an ICU, for monitoring of right ventricular infraction. In such cases, a typical treatment is to load a patient with fluids to increase flow into left ventricle (and prevent shock). However, when the right ventricle starts, fluid load must be reduced. In an exemplary embodiment of the invention, a same sensor is used to detect both situations and optionally automatically control fluid intake and fluid removal (e.g., using dialysis and/or medication), as needed. Optionally, right ventricle activity is detected mechanically. Optionally, ECG signals are used to detect such increased activity as well. Optionally, mechanical signals (e.g., via ultrasound) are used to detect that the left ventricle is experiencing problems due to fluid overload.

Imaging and non-imaging applications

A particular feature of some embodiments of the invention is that the system can be operated in part or in whole and/or data acquired, without reconstructing and/or displaying an image. However, in some embodiments, an image may be reconstructed and/or used together with parameter extraction, for example, as described below.

In an exemplary embodiment of the invention, the data for extracting the cardiac parameters is acquired in a non-imaging mode. Optionally, a user indicates on an image where to sample the parameters, however, both blood and muscle measurements are optionally acquired in a single cardiac cycle. Optionally, the marking on the image is used to delimit ranges of relevant signals.

In an exemplary embodiment of the invention, an image is used to assist in positioning and/or initial aiming of transducers, however, once positioned, no more imaging is used.

In an exemplary embodiment of the invention, a non-imaging mode as described herein is applied using an imaging probe, with the results optionally overlaid on an image.

In some embodiments, an image or map is reconstructed, but not displayed.

Exemplary signal processing

Fig. 9 is a flowchart 900 of a method of signal processing in accordance with an exemplary embodiment of the invention. This method is first described in general and then in greater detail, from some of the acts.

At 902, the geometry of the transducers is optionally determined by interrogation (described below).

At 904, the Doppler (and other) signals are acquired, using, for example, one or three or more transducers (depending on the embodiment). In an exemplary embodiment of the invention, one transducer is provided at or about an apical view, for transmitting and receiving and two or more other receivers are provided at other locations.

Optionally (905) an ECG signal is acquired and may be used for timing of events and/or acquisition.

At 906, the blood signal and muscle signal are optionally separated, for example, using frequency and gain filters.

At 908, the transmitral blood flow signal (E wave) and the early diastolic tissue motion (E'- wave) are identified. Optionally, the shape of the Doppler signal is used for such identification. For example for blood, two sequential positive peaks are found, with the first being E and the second being A. For example for tissue, a complex of a negative peak (A') followed by a positive peak (S') followed by negative peak (E') is found.

At 910, late diastolic transmitral flow (A- wave) , tissue motion (A'- wave) , and systolic tissue motion (S'- wave) are optionally identified. It should be noted that 908 and 910 are optionally carried out before reconstructing any 3D velocity vector, but rather for each transducer. Optionally, 908 and 910 are carried out before reconstruction.

At 912, the signal signature of the mitral valve leaflets is optionally identified.

At 914, peak velocity values for E and E', A, A' and S' are determined. In embodiments where not all these measures are used, them may be not determined (e.g., on the signals), their magnitude not determined and/or no processing (e.g., 3D vector reconstruction) performed for such parameters.

At 916, the distance of the maximum Doppler velocity for each cardiac parameter from the transducer is determined. It should be noted that due to the nature of pulse Doppler there may be more than one range approximately same (e.g., within 10% or 20%) peak velocity value.

At 918, anatomical and/or physiological knowledge is optionally used for timing of events (e.g. using electrical activity from ECG feed), the order of events (e.g. E and E' and A and A' are conjugates), and the relative position (distance) of the structures (e.g. E and E' maxima are typically adjacent to the mitral valve annular ring so that the mitral valve signal signature can be used as a reference). Optionally, this is selected to correspond to a clinical protocol in which the transmitral flow E is measured with sample placed at the level of the mitral leaflets and E' is measured at the level of the annular ring (about 1-2 cm behind the leaflet).

At 920, if there are more than 3 transducers, the system optionally iterates between the sets of transducers to determine which set provides a best configuration. In an exemplary embodiment of the invention, the set is selected according to having a maximum of peak velocity value for an apical transmit/receive transducer and usable reception for at least two other transducers. In an exemplary embodiment of the invention, this act of selecting transducers is not repeated each cycle, but only as needed, as a calibration procedure (e.g., triggered by a user, by a posture sensor, by signal quality deterioration and/or invalid readings), or on a periodic basis. Optionally, a calibration takes several heartbeats and possibly some processing time. Optionally, calibration takes between 10 seconds and 1 minute. Optionally, such a calibration is repeated, for example, in a duty cycle of better than 1:2, 1:4, 1:10, 1:20, 1:40, 1:100 or intermediate values.

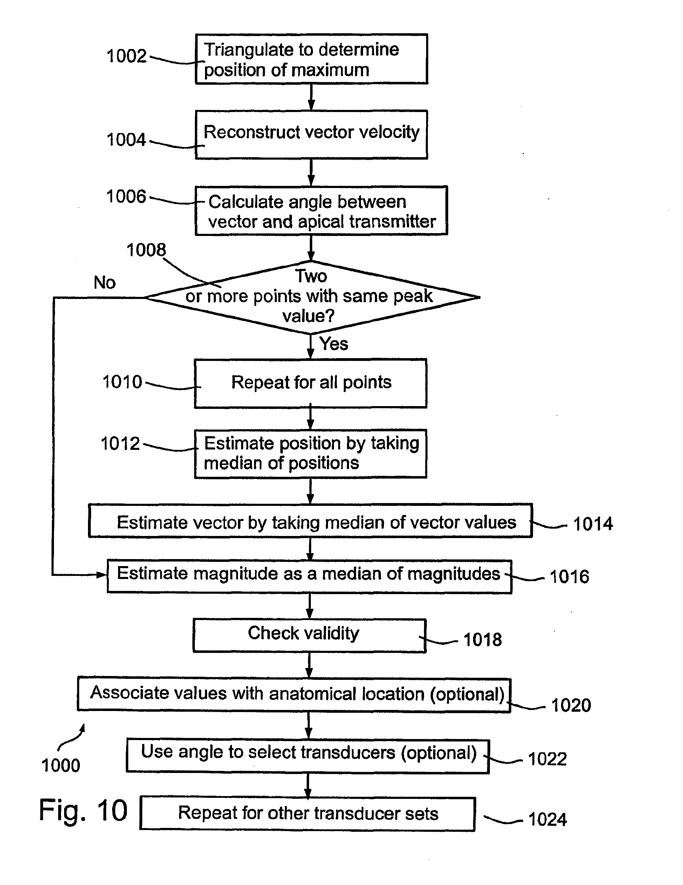

At 922 the location and velocity of tissue structures are optionally determined, as described in Fig. 10.

Referring to Fig. 10, which is a flowchart 100 of a method of structure location and velocity determination in accordance with an exemplary embodiment of the invention.

At 1002, the maximum velocity distance values and the relative positions of the transducers are used to triangulate or otherwise calculate the relative position of the maximum.

At 1004, the velocity vector (Vx, Vy, Vz) at the position of the calculated maximum is reconstructed.

At 1006, the magnitude of the velocity vector and the angle between the vector and the transmitting (e.g., apical transducer) is calculated.

If there are two or more points with the same peak velocity value (1008), the above procedure 1002-1006 is repeated for all points with the same peak velocity value (1010). Then (1012), the position of the structure is estimated using by taking the median or a different function with a preference to high quality signals, apical transducer and/or avoiding extreme values. of the coordinates (i.e. median x, median y and median z.) of the calculated positions. One example of such a function is a weighted average. Another is an average after dropping an extreme value. Another example, is a voting function which goes by majority. Then (1014), the maximum velocity vector components are estimated by taking the median of the calculated velocity vector components coordinates (i.e. median x, median y and median z.) of the calculated positions. Then (1016), the magnitude of maximum velocity is estimated as the median of calculated magnitudes. Simulations of various blood flow profile have shown that the estimated maximum velocity is reliable

At 1018, the validity of the result is optionally checked, for example, using one or more of norms (e.g., velocity, timing, range); anatomy (e.g., position), physiology (e.g., velocity peak and/or direction timing) and/or prior measurements (e.g., second, minutes, hours or longer) of the individual patient. Optionally, a sensor, for example, an accelerometer is used to indicate change in posture or movement which may affect signal. Optionally, if an invalid signal is found and/or if a series of such signals are found, a recalibration and/or reselection of transducers is carried out.

Optionally, determination if a transducer provides a useful signal is based on one or more of a noise level and/or determining if any useful information passes through conditioning filters, such as may be used to detect muscle motion, detect blood motion and/or correct for body motion.

At 1020, the maximum velocity values of tissue are optionally associated with anatomical location (e.g., lateral versus septal), for optional use in clinical protocols that differentiate between the lateral and septal E'.

Optionally, at 1022, the calculated angle is used to select a best configuration for data acquisition.

At 1024, if there are more than 3 transducers, acts 1002-1022 are repeated for one or more other triplets of transducers. Optionally or alternatively, an over-constrained set of equations for more than three transducers is solved, for example, using numerical methods.

In some embodiments of the invention, only muscle Doppler is collected (e.g., E' A' and S' parameters are measured). Since tissue Doppler shifts are smaller by an order of magnitude then those of blood, a smaller PRF can be chosen. Optionally or alternatively, a PRF can be selected so that in a singe cycle, all transducers transmits in turn while all other receivers receive (e.g., for 3, 4, 7 transducers).

Referring back to Fig. 9, at 924 E/E' is optionally estimated. In an exemplary embodiment of the invention, if Valid E and E' are obtained from a triangulation method use the ratio of the calculated magnitude of E and E' from the method of Fig. 10 are used.

In an exemplary embodiment of the invention, if only a valid E' (1018) is obtained from a triangulation method, an obtained E value is corrected to match an angle obtained for E'.

In an exemplary embodiment of the invention, if only a valid E is obtained from a triangulation method, an obtained E' value is corrected using the angle obtained for E.

In an exemplary embodiment of the invention, if triangulation is not valid or if there is only a single apical transducer the ratio of the peak velocities of E and E' of the signal obtained from a transducer at or near apical view is used. It has been surprisingly found that this value is relatively stable for changes in angle.

Exemplary transducer geometry determination and exemplary triangulation and vector extraction of Doppler signals

In an exemplary embodiment of the present invention three or more ultrasound transducers 312 are used to acquire Doppler signals as well as echo signals from the heart covering target volume. The data acquisition comprises an acquisition sequence involving one or more transmitting transducers (that optionally are also receiving) while the other transducers are receiving.

In an exemplary embodiment of the invention, the relative distances 602, 603 and 604 between the transducers (see Fig. 7A) are calculated using a "time of flight" method using their ultrasonic signals for measuring time of flight. In another embodiment the distances 602, 603 and 604 are measured by other means, such as, but not limited to air ultrasound, optical, RF or mechanical means. In another embodiment the distances 602, 603 and 604 are fixed. The calculation of the relative distances 602, 603, and 604 can be done as part of the velocity measurement or as a separate interrogation. Optionally, it is assumed that that the transducers remain fixed during the velocity measurement.

Triangulation, in general, is an established method for calculating the position of an object relative to three or more reference sites. A successful triangulation calculation can be achieved for example if the distances between the object and each of the reference site and the distances between the reference sites are given. Thus for three reference sites (see Fig. 7A) a set of 6 distances (AB, AC, BC; AP, BP, CP} suffices to locate the relative position of object P (up to a sign representing a reflection of a mirror image relative to the reference plane ABC). Measuring the distances (605, 606, and 607) between the object 601 and the reference sites 312 can be done with ultrasound by calculating the time-of- flight between the onset of acoustic pulse and its detection at a sensor located at the site of the object, or if such sensor is not present, by detecting time- of- echo signal from the object and dividing it by two. The conversion to distance is by multiplying the result by the speed of sound (approximately 1540 m/s in body) .

Instead of the three distances between the object and the reference sites {AP, BP, CP) one can use the set of three cross-distances {APB, APC, BPC}, defined as (see Fig. 7B):

$$APB = AP + BP,$$

$$APC = AP + CP,$$

$$BPC = BP + CP. \qquad \text{[Eqs. 1]}$$

The proof is straightforward as the above set of equations can be solved for {AP, BP, CP} yielding:

$$AP = (APB + APC - BPC)/2$$

$$BP = (APB - APC + BPC)/2$$

$$CP = (-APB + APC + BPC)/2. \qquad \text{[Eqs. 2]}$$

Measuring the three cross-distances {APB, APC, BPC}, directly corresponds to measuring the time-of-echo of the cross-beam signal scattered from the object and detected at the face of the adjacent transducers. For example $T_{APB}$ is the time the ultrasound signal travels from transmitter A to object P and from P to until it reaches the face of receiver B. The distance APB is the time $T_{APB}$ multiplied by the speed of sound.

In an exemplary embodiment of the invention, one transducer (A) is sued for both transmitting and receiving and at least two other transducers (B) and (C) are used only for receiving. Hence the three measured distances are

$$APA = 2*AP,$$

$$APB = AP + BP,$$

$$APC = AP + CP. \qquad \text{[Eqs. 1a]}$$

These distances can be mapped back to the standard triangulation set for calculating the relative position of (P).

$$AP = APA/2,$$
$$BP = APB - APA/2$$
$$CP = APC - APA/2. \qquad \text{[Eqs. 2a]}$$

This embodiment potentially has one or more of the following practical advantages:

1. *Fewer pulses, less energy, faster measurement and/or higher repetition rate:* working with three transducers in pulse-echo mode requires three pulses for determining the three distances {AP, BP, CP} whereas the two cross-distances can be determined by only one transmitting pulse, Consequently the acoustic output energy per measurement is reduced, the measurement time is reduced and because fewer pulses are needed per measurement the repetition rate can be increased without aliasing.

2. The distances between the three reference transducers {AB, AC, BC} (602, 603 and 604) are obtained from the first detecting signal across the transducers. This signal corresponds to the shortest distance (straight line) between the transducer. Optionally, this is used instead of the base geometry interrogation (902). Optionally, this is used in transmission cycles where at least two transmitters transmit. Optionally, such a cycle is on the order of milliseconds or tens of milliseconds.

3. *Scalability* - The number of cross-distances increases as $\dfrac{n(n-1)}{2}$ with the total number of transducers n. To determine all these cross-distances n-1 transmitting transducers are needed (optionally with only one transmitting at a time). For example, for a system with 5 transducers {A, B, C, D, and E} of which {A, B, C, and D} are transmitting and receiving and E only receives, the total number of cross-distances is $\dfrac{5(5-1)}{2} = 10$. Similarly, a system with 6 transducers (only 5 of which are transmitting) has a total number of 15 cross-distances. Generally, the number of independent measurements increases quadraticly with the number of total transducers (as n(n-1)/2 - $n^2$). In theory, three transducers suffice to locate an object and therefore the use of additional independent measurements seems redundant. However, in practice noise and other imperfections may lead to large measurement errors. These errors can often be reduced with the use of additional independent measurements so that the overall measurement accuracy is increased. In some cases, the cost of the scalability is aliasing. Because, in general, only one transducer can transmit at a time, the effective PRF is divided by n-1. In muscle only imaging, or where muscle and blood are measured in separate acquisitions, (but optionally same cardiac cycle) this is not generally a problem.

The use of cross-echo triangulation can be extended to Doppler ultrasound. Under the assumption that the object velocity is much lower than the speed of sound it can be shown that the Doppler shift is proportional to the range rate $\dot{R}$ = - dR/dt, the speed of which the object and the observer are closing range. The Doppler shift for the passive case is $\Delta f = f_2 - f_1 = (\dot{R}/c)f_1$ (see pages 453-454 in "Fundamentals of Acoustic" by L. E. Kinsler, A. R. Frey, A. B. Coppens and J. V. Sanders 4th edition John wiley & Sons, Inc.). In the embodiment shown in Fig. 7B, transducer A is transmitting 707 and receiving 710 ultrasonic signals and transducer B and transducer C are receiving the reflected cross-echo signals 709 and 708 respectively. Assuming that transducers A, B, and C are stationary, the Doppler shift is determined by the source frequency $f_0$, the speed of sound $c$, the velocity of the object $v_P$ 701 and its direction relative to transducer A (vector 704) and transducers B (vector 705) and C (vector 706).

Consider the case of A transmitting 707 and C receiving 708, the relative directions is given by $\alpha$ 702 for the orientation of the velocity vector relative to AP (704) (see Fig. 7B). For the case $|v_P| \ll c$ the observed frequency at receiver A is given by

$$f_A = f_0 \left[1 + (v_P / c)\cos\alpha\right]\left[1 + (v_P / c)\cos\alpha\right]$$
$$\cong f_0 \left[1 + 2(v_P / c)\cos\alpha\right], \qquad \text{[Eq. 3a]}$$

and the Doppler Shift as measured at transducer C due to the movement of object P is given by

$$\Delta f^{P}{}_{AA} = (2 v_P f_0 / c)\cos\alpha. \qquad [\text{Eq. 4a}]$$

Consider the case of A transmitting 707 and C receiving 708, the relative directions are given by $\alpha$ 702 and $\gamma$ 703 for the orientation of the velocity vector relative to AP (704) and CP (705), respectively (see Fig. 7B). For the case $|v_P| << c$ the observed frequency at receiver C is given by ,

$$f_C = f_0[1 + (v_P / c)\cos\alpha][1 + (v_P / c)\cos\gamma]$$
$$\cong f_0[1 + (v_P / c)(\cos\alpha + \cos\gamma)] \qquad , \qquad [\text{Eq. 3}]$$

and the Doppler Shift as measured at transducer C due to the movement of object P is given by

$$\Delta f^{P}{}_{AC} = (v_P f_0 / c)(\cos\alpha + \cos\gamma). \qquad [\text{Eq. 4}]$$

The scalar product of any two vectors $\vec{a}$ and $\vec{v}$ is defined as

$$\vec{a} \cdot \vec{v} = (a \times v)\cos\theta_{av}. \qquad [\text{Eq. 5}]$$

It follows that $v_P/c \cos\alpha = \hat{A}_P \cdot \vec{v}_P/c$ where $\hat{A}_P$ is a unit vector in the direction PA 704. Similarly $v_P/c \cos\gamma = \hat{C} \cdot \vec{v}_P/c$ where $\hat{C}_P$ is a unit vector in the direction PC 705. The Doppler shift can be expressed as

$$\Delta f^{P}{}_{AC} = f_0 \hat{A}_P \cdot \vec{v}_P / c + f_0 \hat{C}_P \cdot \vec{v}_P / c = f_0(\hat{A}_P + \hat{C}_P) \cdot \vec{v}_P / c \qquad [\text{Eq. 6}]$$

Similarly, for the other cross- echoes the Doppler Shifts are given by

$$\Delta f^{P}{}_{AB} = f_0(\hat{A}_P + \hat{B}_P) \cdot \vec{v}_P / c \text{ and } \Delta f^{P}{}_{AA} = 2 f_0(\hat{A}_P) \cdot \vec{v}_P / c \quad [\text{Eqs. 7}]$$

The Doppler shift $\Delta f^{P}{}_{AA}$ will appear in the time trace of the echo channel AA at a time equal to 2AP/c. The Doppler shift $\Delta f^{P}{}_{AB}$ will appear in the time trace of the cross-echo channel AB at a time equal to (AP+BP)/c. Similarly, the Doppler shift $\Delta f^{P}{}_{AC}$ will appear in the time trace of the cross-echo channels AC at a time equal to (AP+CP)/c.

The linear transformation Eq. 2 can be employed to Eqs. 6 and 7 yielding

$$\Delta f^P{}_A = APA/2$$
$$= \Delta f^P{}_{AA}/2 \quad ,$$
$$= f_0 \hat{A}_P \cdot \vec{v}_P / c$$

$$\Delta f^P{}_B = (APB - APA/2)$$
$$= (\Delta f^P{}_{AB} + \Delta f^P{}_{AA}/2)$$
$$= f_0(\hat{A}_P + \hat{B}_P) \cdot \vec{v}_P / c - f_0 \hat{A}_P \cdot \vec{v}_P / c \quad ,$$
$$= f_0 \hat{B}_P \cdot \vec{v}_P / c$$

$$\Delta f^P{}_C = f_0 \hat{C}_P \cdot \vec{v}_P / c . \qquad \text{[Eqs. 8]}$$

Eq. 4 and Eq. 5 can be used to derive the standard Doppler echoes (with three transmitting/receiving transducers) yielding

$$\Delta f^P{}_{AA} = 2 f_A \hat{A}_P \cdot \vec{v}_P / c$$
$$\Delta f^P{}_{BB} = 2 f_B \hat{B}_P \cdot \vec{v}_P / c \qquad \text{[Eqs. 9]}$$
$$\Delta f^P{}_{CC} = 2 f_C \hat{C}_P \cdot \vec{v}_P / c$$

It follows that

$$\Delta f^P{}_{AA} = 2 \Delta f^P{}_A$$
$$\Delta f^P{}_{BB} = 2 \Delta f^P{}_B \qquad \text{[Eqs. 10]}$$
$$\Delta f^P{}_{CC} = 2 \Delta f^P{}_C$$

In an exemplary embodiment of the invention, the coordinates of P are found via a standard triangulation method using the set of distances {AB, AC, BC; AP, BP, CP}. The unit vectors $\hat{A}_P$, $\hat{B}_P$ and $\hat{C}_P$ are calculated according to:

$$\hat{A}_P = \frac{\vec{P} - \vec{T}_A}{\left| \vec{P} - \vec{T}_A \right|}$$

$$\hat{B}_P = \frac{\vec{P} - \vec{T}_B}{\left| \vec{P} - \vec{T}_B \right|} , \text{[Eqs. 10a]}$$

$$\hat{C}_P = \frac{\vec{P} - \vec{T}_C}{\left| \vec{P} - \vec{T}_C \right|}$$

where P is the radius vector to point P, and $\overline{T}_A$, $\overline{T}_B$ and $\overline{T}_C$ are radius vector to transducers A, B and C, respectively.

In an exemplary embodiment of the invention, the Doppler shifts $\Delta f^P{}_{AA}$, $\Delta f^P{}_{AB}$ and $\Delta f^P{}_{AC}$ are measured and the velocity vector $\overline{v}_P$ is extracted by solving the set of Eqs. 8, an exemplary solution of which is given below.

First the vectors $\hat{A}_P$, $\hat{B}_P$, $\hat{C}_P$, $\overline{v}_P$ are expressed by their Cartesian components:

$$\hat{A}_P = \{A_x^P, A_y^P, A_z^P\}/AP$$

$$\hat{B}_P = \{B_x^P, B_y^P, B_y^P\}/BP$$

$$\hat{C}_P = \{C_x^P, C_y^P, C_z^P\}/CP$$

$$\bar{v}_P = \{V_x^P, V_y^P, V_z^P\} \qquad\qquad \text{[Eqs. 11]}$$

Solving the set of equations 8 for $\bar{v}_P$ yields

$$v_x^P = \frac{\left(B_z^P C_y^P - B_y^P C_z^P\right)AP\Delta f_A^P + \left(A_y^P C_z^P - A_z^P C_y^P\right)BP\Delta f_B^P + \left(A_z^P B_y^P - A_y^P B_z^P\right)CP\Delta f_C^P}{A_z^P B_y^P C_x^P - A_y^P B_z^P C_x^P - A_z^P B_x^P C_y^P + A_x^P B_z^P C_y^P + A_y^P B_x^P C_z^P - A_x^P B_y^P C_z^P}$$

$$v_y^P = \frac{\left(B_z^P C_x^P - B_x^P C_z^P\right)AP\Delta f_A^P + \left(A_x^P C_z^P - A_z^P C_x^P\right)BP\Delta f_B^P + \left(A_z^P B_x^P - A_x^P B_z^P\right)CP\Delta f_C^P}{-A_z^P B_y^P C_x^P + A_y^P B_z^P C_x^P + A_z^P B_x^P C_y^P - A_x^P B_z^P C_y^P - A_y^P B_x^P C_z^P + A_x^P B_y^P C_z^P}$$

$$v_z^P = \frac{\left(B_y^P C_x^P - B_x^P C_y^P\right)AP\Delta f_A^P + \left(A_x^P C_y^P - A_y^P C_x^P\right)BP\Delta f_B^P + \left(A_y^P B_x^P - A_x^P B_y^P\right)CP\Delta f_C^P}{A_z^P B_y^P C_x^P - A_y^P B_z^P C_x^P - A_z^P B_x^P C_y^P + A_x^P B_z^P C_y^P + A_y^P B_x^P C_z^P - A_x^P B_y^P C_z^P}$$

[Eqs. 12].

The magnitude of the velocity vector is calculated by:

$$V_p = \sqrt{V_x^2 + V_y^2 + V_z^2} \qquad \text{[Eq.13]}.$$

And the angle between the transmitter A and the velocity vector is given by:

$$\alpha = Cos^{-1}\left(\hat{A}_P \cdot \bar{V}_P / V_p\right) \qquad \text{[Eq. 14]}$$

Similarly, it is possible to employ (not shown here) triangulation methods to either a set of three Doppler echoes, or a set of three Doppler cross- echoes, or a set of one Doppler echo and two cross- echoes in order to reconstruct the original velocity vector of an object.

Peak velocity determination

In an exemplary embodiment of the invention, the extreme (minimum or maximum) peak velocity (Doppler shift) is calculated for each of the receiving channels, along with the distance associated with this maximum. Optionally, these distances are used by a triangulation method for estimating the location of the maximum signal. Then, the peak velocity vector is constructed using Eqs. 12. The magnitude of the vector is calculated according to Eq. 13 and the angle $\alpha$ between the transmitter and the velocity vector is calculated using Eq. 14.

In another embodiment the projection of the velocity vector on any arbitrary axis is calculated. This may be useful, for example, if a measurement needs only a longitudinal component, for example, of the muscle movement. For example, the component of tissue velocity in the direction of the blood flow can be thus calculated.

Application to broad-beam transducers

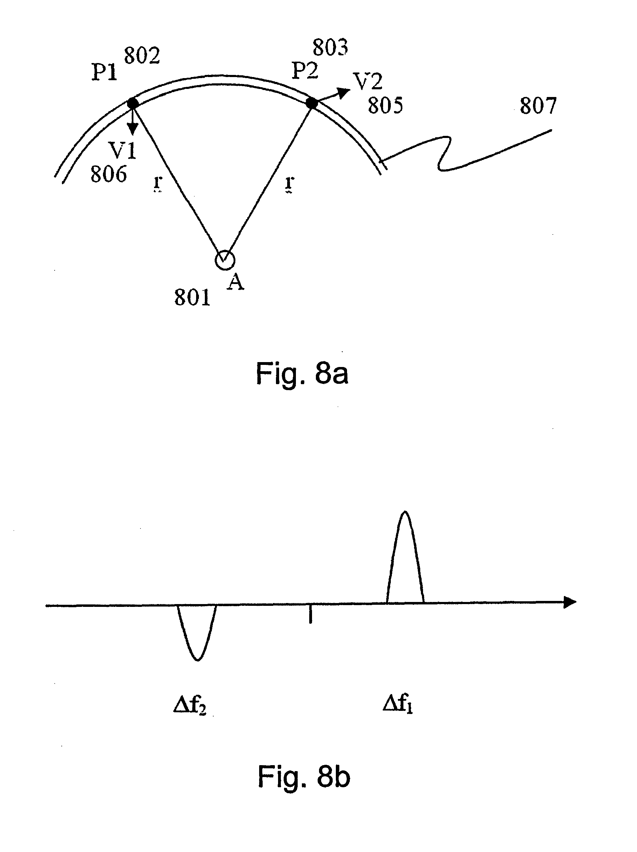

In general, an ultrasonic transducer (as any radar-type of instrumentation) associates range to the time elapsed between events (e.g. transmission and reception) multiplied by the speed of sound. However, in the case of broad-beam profile the time trace signal is a sum of contributions originating from multiple equidistant points generally arranged on a section of a sphere surface associated with the corresponding range. Similarly, the Doppler spectrum is a superposition of all Doppler shifts associated with moving objects in the equidistance sphere surface. For example, consider the case depicted in Fig. 8A of two distinct objects P1 (802) and P2 (803) with two different velocity vectors V1 (806) and V2 (805), respectively. The objects are located in the equidistance plane 807 at the same distance (r) from the transducer A (801). The Doppler spectrum would appear as the superposition $\Delta f_1 + \Delta f_2$ (Fig. 8B). Thus, the Doppler signal is degenerate (i.e. has more than one frequency shift for a given range). This can lead to ambiguities when reconstructing the 3D velocity vector map, for example, more than one velocity can be associated with the same volume element. This ambiguity generally renders the use of non-focused transducers impractical for a reliable reconstruction of a 3D velocity vector map. However, it has been surprisingly found that under certain conditions, for example when there is a single dominant peak velocity inside the region of interest at the time of measurement, the maximum peak velocity vector can still be reliably calculated. In particular, for cardiac blood and tissue Doppler signals this method is found to be very reliable for deriving the magnitude of the peak velocity vector, and to a lesser extent the exact location of moving object.

In an exemplary embodiment of the invention, more than three transducers (for example, four, five or six or more), of which some or all are broad-beam are used. Not all the transducers need to have a same beam breadth. Optionally, assuming a total of N transducers, the velocity vector of each volume element P is constructed for up to

$$\binom{N}{3}$$ possible triangulation bases. The construction is done in similar fashion to the described above. As described below the redundancy of measurements for the same volume element can assist in lifting degeneracies caused by the non-focality of the transducers.

As indicated above this ambiguity can be lifted if more than one base is used for reconstructing the vector map. Consider for example a five transducers system (A, B, C, D and E) in which the Doppler time trace of A at point P is degenerate while the Doppler time traces of transducers B, C, D, and E at point P are not degenerate. In $\binom{4}{3} = 4$

(out of the total of $\binom{5}{3} = 10$) velocity vector triangulation bases the degeneracy associated with time trace of A may lead to erroneous results. However, in the remaining 6 triangulation bases the velocity vector is correctly reconstructed. Optionally, a simple majority principle is used for error correction.

In an exemplary embodiment of the invention, even if there are bad channels, a useful triangulation is achieved by assuming there is a majority of "correct" triangulations and selecting these using a type of "majority rule".

Single transducer system,

In an exemplary embodiment of the invention, a single transducer (or pair of a transmitter and a receiver) are used to obtain useful data, for example, if the transducer is apical. It has been surprisingly found that the ratio E/E' is relatively robust to off-axis aiming, for example, when the transducer view is at an angle (volumetric) of 5 degrees, 10 degrees, 20 degrees, 30 degrees, 40 degrees or intermediate angles relative to line of motion of the peak blood and tissue movement vector.

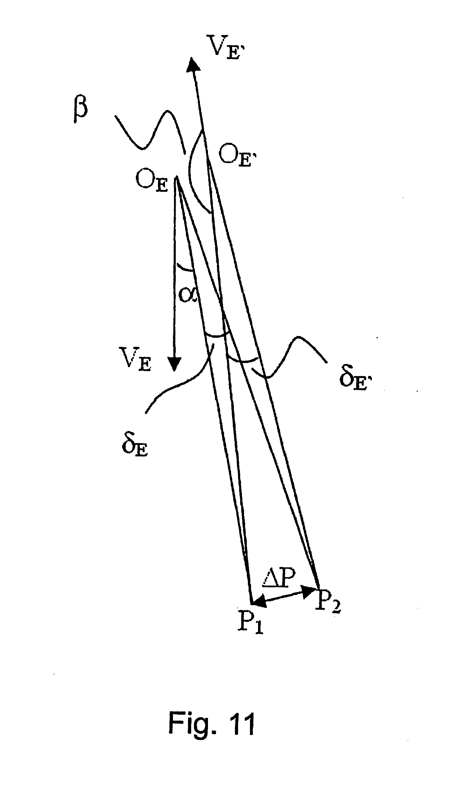

Following is a 2D analysis, which is shown for simplicity, but it is believed that similar results are obtained for 3D. Fig. 11 shows a transducer aimed at a heart, as follows:

$V_E$ is the transmitral blood flow velocity vector associated with E.
$V_{E'}$ is the tissue velocity vector associated with E'.
$O_E$ is the origin of the velocity $V_E$.
$O_{E'}$ is the origin of the velocity $V_{E'}$.
P1 is a first position of a single transducer (close or at apical view)
P2 is another position for a single transducer displaced by $\Delta P$ from P1
$\alpha$ is the angle between line of sight of transducer P1 (OP1) and $V_E$
$\beta$ is the angle between line of sight of transducer P1 (OP1) and $V_{E'}$

$\delta_E$ and $\delta_{E'}$ are the angle changes caused by moving from OP1 to OP2.

$\delta_E \approx \delta_{E'} = \delta$ : This follows from the assumption that the distance ($\Delta O$) between $O_E$ and $O_{E'}$ is much smaller than OP1 and OP2 (typically OP1 and OP2 ~10 cm and $\Delta O$~1-2 cm).

Under the above assumptions the measured ratio E/E' at position P1 is given by:

$$\frac{E_{P1}}{E'_{P1}} = \frac{V_E \cos\alpha}{V_{E'} \cos\beta}$$

whereas the ratio measured at P2 is given by

$$\frac{E_{P2}}{E'_{P2}} = \frac{V_E \cos(\alpha + \delta_E)}{V_{E'} \cos(\beta + \delta_{E'})} \approx \frac{V_E \cos(\alpha + \delta)}{V_{E'} \cos(\beta + \delta)}$$

The relative error R between these two measurements is given by

$$R \equiv \left(\frac{E_{P2}}{E'_{P2}}\right) \bigg/ \left(\frac{E_{P1}}{E'_{P1}}\right) = \frac{\cos(\alpha + \delta)\cos\beta}{\cos(\beta + \delta)\cos\alpha}$$

For the special case where the blood and tissue velocities have opposite direction ($\alpha - \beta = \pi$) the ratio is 1 regardless of the value of $\delta$.

Generally $\alpha - \beta = \pi + \gamma$, where $\gamma$ is a small angle (an assumption based on physiology), and if also assuming that $\delta$ is small, the ratio defining R expanded in a series to first order is:

$$R \cong 1 + \gamma\delta + \gamma\delta \tan^2\alpha$$

If P1 is initially placed at or near apical view then $\alpha$ is a small angle, so that the ratio R differs from 1 by $\gamma\delta$. $\delta$ and $\Delta P$ are optionally related by the cosine theorem:

$$\Delta P^2 \approx OP1^2 + OP2^2 - 2OP2 \times OP2 \times \cos\delta$$

In an exemplary embodiment, OP1 and OP2 both equal 10 cm, and the distance of P2 from the "ideal" apical view is 3 cm so that $\delta$ is 0.3 (17 degrees), and $\gamma$ is 0.16 (10 degrees) so that according to the above equations, the deviation of the ratio from 1 is by less than 5%.

The above 2D analysis represents a worst case scenario. In a full 3D analysis, the velocity vectors, their origins and the transducer's position are not in the same plane. In 3D, the value $\delta$ is smaller (factored by cos of an angle) so that the actual deviation of the ratio R from unity is expected to be lower.

In summary, based on the fact the both E and E' are scaled by a cosine of a similar angle, their ratio can be relatively reliably measured with a single transducer (e.g., broad beam) even if the transducer is positioned in a suboptimal view point.

Exemplary settings

In an exemplar embodiment of the invention, settings are selected to view blood velocities of up to 1.7 m/s. Above that value, velocities are often caused by pathological mitral stenosis. Assuming heart is at a depth of ~10 cm, the depth settings should be ~15 cm. At 2 MHz, this means that a PRF of 5Khz, provides 15 cm penetration. Settings are generally -2 m/s for maximum velocity (blood). Lowest measurable speed is close to zero, depending on DC filters, for example. In an exemplary embodiment of the invention, the numbers are changed to match the expected window, determined for example, by imaging or by a previous measurement, optionally with a lower resolution but greater penetration. In general, the number of cycles in a pulsed window depends on signal to noise ratio, depth resolution and acoustic output. In an exemplary embodiment of the invention, 2-16 (optionally 8) cycles per pulse are used.

In an exemplary embodiment of the invention, the system settings are selected so that less than 50%, less than 30%, less than 25%, less than 15% of the heart is within range and angle windows.

Depending on the clinical protocol, a user may select to selectively view septal or lateral muscle motion. Optionally, a user views an average. Optionally, a lateral signal is preferentially detected, as its peak value is larger.

It should be noted that other transducers, frequencies and/or shapes may be used, as known in the art of cardiac Doppler signal detection for example.

Example

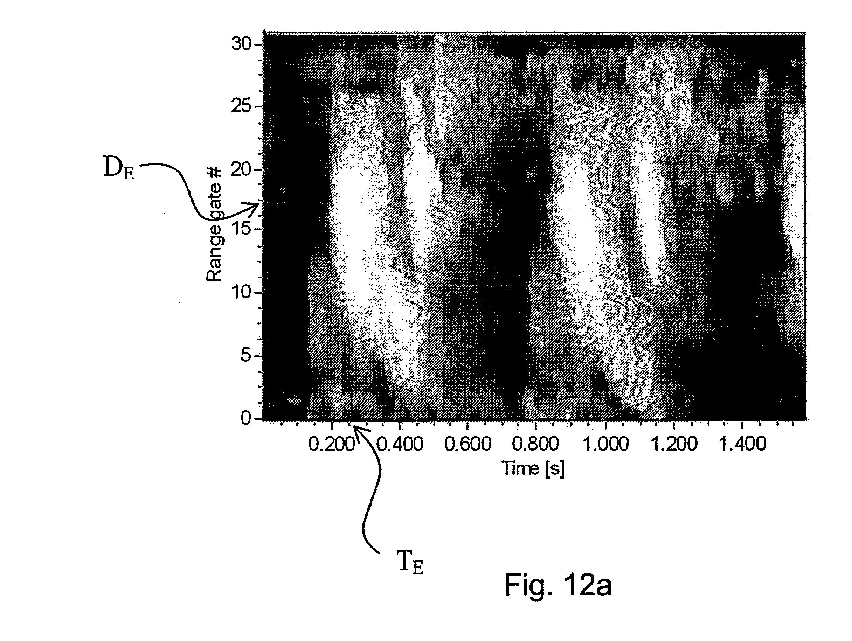

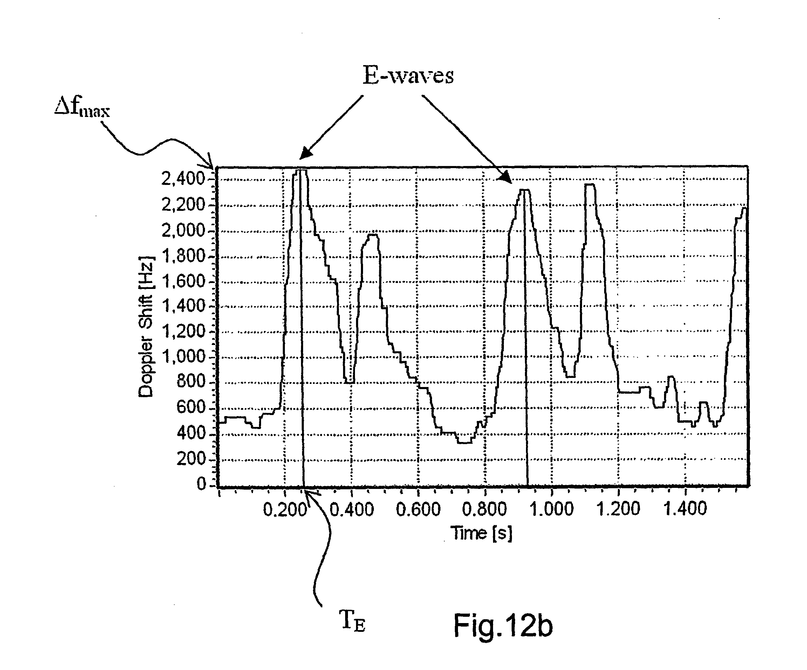

Figs. 12A and 12B illustrate results of measurements, in accordance with an exemplary embodiment of the invention. Fig. 12A shows a typical 2D time range Doppler signal obtained by one of the receiving transducers. The bottom axis is the time in seconds and the left axis is the range gate number of the multigate detection. The 32 gates ranging from 30 mm for gate #0 in steps of 3.75 mm. The brighter intensity in the grayscale display reflects the higher Doppler shifts. The time of the e-wave peak ($T_E$) velocity and the gate it was obtained (#17) which corresponds to a distance of $D_E$ =93.75 mm, are indicated.

Fig. 12B shows a time velocity Doppler trace (after edge detection) of the maximum peak velocity as recorded at gate #17. Two E-waves are clearly seen and the maximum Doppler shifts value of 2480 Hz associated with the first E-wave is indicated. The data was acquired with a 2MHz probe at a PRF of 5 kHz.

As noted above, similar data can be obtained for the other two channels (not shown). The triangulation algorithm further uses the 3 distances $D_E$ obtained in the three channels at time $T_E$, together with the coordinates of transmitters to find the origin of the Doppler signal (P vector). The unit vectors $\hat{A}_P$, $\hat{B}_P$ and $\hat{C}_P$ are calculated according to Eq 10a and the peak velocity vector is reconstructed using Eq. 12.

**General**

Various designs for ultrasonic probes and ultrasonic imaging systems are known in the art. It should be appreciated that various ones of the methods described herein may be adapted for such probes and/or systems, in accordance with exemplary embodiments of the invention.

While the above description has focused on aortic and mitral valves, the methods and apparatus may be applied, for example, to detect and measure parameter at or about the tri-cuspid and pulmonary vales, for example, to measure trans-tricuspid regurgitation and/or velocity (Blood, tissue) that indicate pulmonary diastolic pressures.

While the above description has focused on pulsed ultrasound (which may be more amenable to various types of digital processing), it may also be applied to continuous wave ultrasound, especially if a distance is determinable.

In an exemplary embodiment of the invention, beam steering is sued (with CW or pulsed) to detect a location of a peak value. Once that value is found, a narrow beam may be aimed at that location to detect relevant parameters. In some cases, two beams would be used, for different tissues in the heart. Optionally, a scan is made periodically, to confirm that the beam is still aimed at a peak location. Such scan may be made, for example, every few minutes or seconds, or if the determined values change significantly or is a noise level in the signal goes up or signal quality otherwise deteriorates.

It will be appreciated that the above described probes, systems and methods of cardiac measurement may be varied in many ways, including, omitting or adding steps, changing the order of steps and the types of devices used. In addition, a multiplicity of various features, both of method and of devices have been described. In some embodiments mainly methods are described, however, also apparatus adapted for performing the methods are considered to be within the scope of the invention. It should be appreciated that different features may be combined in different ways. In particular, not all the features shown above in a particular embodiment are necessary in every similar embodiment of the invention. Further, combinations of the above features are also considered to be within the scope of some embodiments of the invention. Also within the scope of the invention are kits which include probes, attachment means, instructions for use and/or systems. Also, within the scope is hardware, software and computer readable-media including such software which is used for carrying out and/or guiding the steps described herein, such as probe control and/or data analysis. Section headings are provided for assistance in navigation and should not be considered as necessarily limiting the contents of the section. When used in the following claims, the terms "comprises", "includes", "have" and their conjugates mean "including but not limited to". It should also be noted that the device is suitable for both male and female, with male pronouns sometimes being used for convenience.

It will be appreciated by a person skilled in the art that the present invention is not limited by what has thus far been described. Rather, the scope of the present invention is limited only by the following claims.

**Claims**

1. A method of cardiac monitoring, comprising:

   a) aiming (604, 606), with a low angular precision, a non-imaging ultrasonic sensor (312) at the heart to receive a Doppler signal from said heart;
   b) automatically identifying (608, 610) a spatially specific tissue in said heart by said sensor based on a signature pattern in said received signal;
   c) extracting (612) a value of a cardiac parameter associated with said specific tissue using the signal received by said sensor (312);
   d) continuing cardiac monitoring by:

      i) receiving a further signal from said specific tissue;
      ii) identifying said specific tissue in said further signal; and
      iii) extracting a further value of said cardiac parameter from a signal detected by said sensor (312); and

   e) repeating d) for at least 10 minutes.

2. A method according to claim 1, comprising periodically verifying that said non-imaging sensor (312) is aimed at said specific tissue.

3. A method according to claim 1 or claim 2, wherein extracting comprises extracting both a tissue velocity and a blood velocity in a same cardiac cycle.

4. A method according to any of the precedin claims, wherein aiming comprises aiming a plurality of spaced-apart non-imaging ultrasonic sensors and wherein d) comprises using a different sensor than the one used for a).

5. A method according to any of the preceding claims, wherein aiming comprises aiming a plurality of spaced-apart non-imaging ultrasonic sensors and wherein c) comprises extracting using signals from multiple of said sensors to reconstruct a vector.

6. A method according to any of the preceding claims, in which at least one parameter of a patient is tracked on a beat-to-beat level in a continuous manner, said at least one parameter includes one or more of E, E', E/E', S', Stroke Volume (SV) and Cardiac output (CO).

7. A method according to any of the preceding claims, wherein said monitoring is ambulatory.

8. A cardiac monitor, comprising:

   a) a body (302) suitable for implanting, holding by hand, attaching to a human or wearing by a human;
   b) at least one ultrasonic sensor (312) adapted to acquire a Doppler signal from the heart; and
   c) circuitry (304), in said body, and adapted to:

      automatically identify (608, 610) a spatially specific tissue in said heart by said sensor based on a signature pattern in said received signal;
      extract (612) a value of a cardiac parameter associated with said specific tissue using the signal acquired by said sensor (312);
      continue said cardiac monitoring by:

         i) receiving a further signal from said specific tissue;
         ii) identifying said specific tissue in said further signal; and
         iii) extracting a further value of said cardiac parameter from a signal acquired by said sensor (312).

**9.** A monitor according to claim 8, formed as part of or coupled to a pacemaker, a stress test monitor, a fluid control system or a halter.

**10.** A monitor according to claim 8 or claim 9, comprising a memory (305) storing therein a baseline value for said cardiac parameter, and wherein said circuitry (304) is configured to compare signals received over a period of over a few minutes with said stored baseline.

**11.** A monitor according to any of claims 8-10, wherein said circuitry (304) is configured for one or both of selecting a best measurement over several heartbeats and for averaging a measurement over several heart beats.

**12.** A monitor according to any of claims 8-11, wherein said circuitry (304) is configured to log measurements of said value.

**13.** A monitor according to any of claims 8-11, wherein said circuitry (304) is battery powered and is configured to generate a wireless alert in response to said value.

**14.** A monitor according to any of claim 8-13, wherein said monitor is configured for monitoring fluid balance.

**15.** A monitor according to any of claims 8-14, wherein said monitor is configured for monitoring myocardial function.

**16.** A monitor according to any of claims 8-15, wherein said monitor is configured for monitoring changes in ejection fraction.


**Patentansprüche**

**1.** Verfahren zur Herzüberwachung, umfassend:

a) Ausrichten (604, 606) eines nicht bildgebenden Ultraschallsensors (312) mit einer niedrigen Winkelpräzision auf das Herz, um ein Dopplersignal von dem Herz zu empfangen;
b) automatisches Identifizieren (608, 610) eines räumlich spezifischen Gewebes in dem Herz durch den Sensor, basierend auf einem Signaturmuster in dem empfangenen Signal;
c) Extrahieren (612) eines Werts eines Herzparameters, der mit dem spezifischen Gewebe verknüpft ist, unter Verwendung des Signals, das von dem Sensor (312) empfangen wurde;
d) Fortführen der Herzüberwachung durch:

i) Empfangen eines weiteren Signals von dem spezifischen Gewebe;
ii) Identifizieren des spezifischen Gewebes in dem weiteren Signal; und
iii) Extrahieren eines weiteren Werts des Herzparameters aus einem Signal, das von dem Sensor (312) erfasst wird; und

e) Wiederholen von d) für mindestens 10 Minuten.

**2.** Verfahren nach Anspruch 1, umfassend periodisches Überprüfen, dass der nicht bildgebende Sensor (312) auf das spezifische Gewebe ausgerichtet ist.

**3.** Verfahren nach Anspruch 1 oder 2, wobei das Extrahieren ein Extrahieren sowohl einer Gewebegeschwindigkeit als auch einer Blutgeschwindigkeit in einem gleichen Herzzyklus umfasst.

**4.** Verfahren nach einem der vorhergehenden Ansprüche, wobei das Ausrichten das Ausrichten von mehreren voneinander entfernten nicht bildgebenden Ultraschallsensoren umfasst, und wobei d) das Verwenden eines anderen Sensors als den umfasst, der für a) verwendet wurde.

**5.** Verfahren nach einem der vorhergehenden Ansprüche, wobei das Ausrichten das Ausrichten von mehreren voneinander entfernten nicht bildgebenden Ultraschallsensoren umfasst, und wobei c) das Extrahieren unter Verwendung von Signalen von mehreren der Sensoren umfasst, um einen Vektor zu rekonstruieren.

**6.** Verfahren nach einem der vorhergehenden Ansprüche, wobei mindestens ein Parameter eines Patienten auf einem Schlag-zu-Schlag-Niveau in einer kontinuierlichen Weise verfolgt wird, wobei der mindestens eine Parameter eines oder mehrere von E, E', E/E', S', Schlagvolumen (SV) und Herzleistung (CO) umfasst.

7. Verfahren nach einem der vorhergehenden Ansprüche, wobei das Überwachen ambulant ist.

8. Herzmonitor, umfassend:

a) ein Gehäuse (302), das zum Implantieren, zum Halten mit der Hand, zum Anbringen an einem Menschen oder zum Tragen durch einen Menschen geeignet ist;
b) mindestens einen Ultraschallsensor (312), der dazu eingerichtet ist, ein Dopplersignal von dem Herz zu erhalten; und
c) eine Schaltung (304) in dem Gehäuse, und eingerichtet zum:

automatischen Identifizieren (608, 610) eines räumlich spezifischen Gewebes in dem Herz durch den Sensor, basierend auf einem Signaturmuster in dem empfangenen Signal;
Extrahieren (612) eines Werts eines Herzparameters, der mit dem spezifischen Gewebe verknüpft ist, unter Verwendung des Signals, das von dem Sensor (312) erhalten wurde;
Fortführen der Herzüberwachung durch:

i) Empfangen eines weiteren Signals von dem spezifischen Gewebe;
ii) Identifizieren des spezifischen Gewebes in dem weiteren Signal; und
iii) Extrahieren eines weiteren Werts des Herzparameters aus einem Signal, das von dem Sensor (312) erhalten wurde.

9. Überwachungseinrichtung nach Anspruch 8, gebildet als Teil von oder gekoppelt mit einem Schrittmacher, einer Stresstestüberwachung, einem Fluidsteuerungssystem oder einem Halfter.

10. Überwachungseinrichtung nach Anspruch 8 oder Anspruch 9, umfassend einen Speicher (305), der darin einen Basiswert für den Herzparameter speichert, und wobei die Schaltung (304) dazu konfiguriert ist, Signale mit dem gespeicherten Basiswert zu vergleichen, die über eine Zeitspanne von mehr als wenigen Minuten empfangen wurden.

11. Überwachungseinrichtung nach einem der Ansprüche 8 - 10, wobei die Schaltung (304) dazu konfiguriert ist für Auswählen einer besten Messung über mehrere Herzschläge und/oder Mitteln einer Messung über mehrere Herzschläge.

12. Überwachungseinrichtung nach einem der Ansprüche 8 - 11, wobei die Schaltung (304) dazu konfiguriert ist, Messungen des Werts aufzuzeichnen.

13. Überwachungseinrichtung nach einem der Ansprüche 8 - 11, wobei die Schaltung (304) batteriebetrieben ist und dazu konfiguriert ist, einen drahtlosen Alarm in Reaktion auf den Wert zu erzeugen.

14. Überwachungseinrichtung nach einem der Ansprüche 8 - 13, wobei die Überwachungseinrichtung dazu konfiguriert ist, eine Fluidbalance zu überwachen.

15. Überwachungseinrichtung nach einem der Ansprüche 8 - 14, wobei die Überwachungseinrichtung dazu konfiguriert ist, eine Myokard-Funktion zu überwachen.

16. Überwachungseinrichtung nach einem der Ansprüche 8 - 15, wobei die Überwachungseinrichtung dazu konfiguriert ist, Änderungen in der Auswurffraktion zu überwachen.

**Revendications**

1. Procédé de surveillance cardiaque consistant à :

a) envoyer (604, 606), avec une précision angulaire faible, un capteur à ultrasons ne produisant pas d'images (312) vers le coeur afin de recevoir un signal Doppler dudit coeur ;
b) identifier automatiquement (608, 610) un tissu spécifique dans l'espace dans ledit coeur avec ledit capteur, sur la base d'un modèle de signature dans ledit signal reçu ;

c) extraire (612) une valeur d'un paramètre cardiaque associé audit tissu spécifique en utilisant le signal reçu par ledit capteur (312) ;

d) continuer la surveillance cardiaque en :

i) recevant un signal supplémentaire dudit tissu spécifique ;

ii) identifiant ledit tissu spécifique dans ledit signal supplémentaire ; et

iii) extrayant une valeur supplémentaire dudit paramètre cardiaque à partir d'un signal détecté par ledit capteur (312) ; et

e) répéter d) pendant environ 10 minutes.

2. Procédé selon la revendication 1, consistant à vérifier périodiquement que ledit capteur produisant pas d'images (312) est destiné audit tissu spécifique.

3. Procédé selon la revendication 1 ou la revendication 2, où l'extraction consiste à extraire à la fois une vitesse du tissu et une vitesse du sang pendant un même cycle cardiaque.

4. Procédé selon l'une quelconque des revendications précédentes, où l'envoi comprend l'envoi d'une pluralité de capteurs espacés à ultrasons ne produisant pas d'images et où d) comprend l'utilisation d'un capteur différent de celui utilisé pour a).

5. Procédé selon l'une quelconque des revendications précédentes où l'envoi comprend l'envoi d'une pluralité de capteurs espacés à ultrasons ne produisant pas d'images et où c) comprend l'extraction en utilisant des signaux provenant d'une multiplicité desdits capteurs pour reconstruire un vecteur.

6. Procédé selon l'une quelconque des revendications précédentes, où au moins un paramètre d'un patient est suivi grâce à ses battements cardiaques de manière continue, ledit au moins un paramètre comprend un ou plusieurs éléments parmi E, E', E/E', S', Débit systolique (DS) et Débit cardiaque (DC).

7. Procédé selon l'une quelconque des revendications précédentes, où la surveillance se déroule de manière ambulatoire.

8. Moniteur cardiaque comprenant :

a) un corps (302) adapté à une implantation, une tenue à la main, une fixation à un être humain ou un port par un être humain ;

b) au moins un capteur à ultrasons (312) adapté pour recevoir un signal Doppler du coeur ; et

c) un circuit (304), dans ledit corps, et adapté pour :

identifier automatiquement (608, 610) un tissu spécifique dans l'espace dans ledit coeur avec ledit capteur, sur la base d'un modèle de signature dans ledit signal reçu ;

extraire (612) une valeur d'un paramètre cardiaque associé audit tissu spécifique en utilisant le signal reçu par ledit capteur (312),

continuer ladite surveillance cardiaque en :

i) recevant un signal supplémentaire dudit tissu spécifique ;

ii) identifiant ledit tissu spécifique dans ledit signal supplémentaire ; et

iii) extrayant une valeur supplémentaire dudit paramètre cardiaque à partir d'un signal détecté par ledit capteur (312).

9. Moniteur selon la revendication 8, faisant partie de ou couplé à un pacemaker, un moniteur de test de stress, un système de contrôle des fluides ou un licou.

10. Moniteur selon la revendication 8 ou la revendication 9, composé d'une mémoire (305) stockant une valeur de base dudit paramètre cardiaque et où ledit circuit (304) est configuré pour comparer les signaux reçus pendant quelques minutes avec la valeur de base stockée.

**11.** Moniteur selon l'une quelconque des revendications 8 à 10, où ledit circuit (304) est configuré pour choisir une meilleure mesure après plusieurs battements cardiaque et/ou faire la moyenne des mesures après plusieurs battements cardiaques.

**12.** Moniteur selon l'une quelconque des revendications 8 à 11, où ledit circuit (304) est configuré pour enregistrer les mesures de ladite valeur.

**13.** Moniteur selon l'une quelconque des revendications 8 à 11, où ledit circuit (304) est alimenté par piles et configuré pour générer une alerte sans fil en réponse à ladite valeur.

**14.** Moniteur selon l'une quelconque des revendications 8 à 13, où ledit moniteur est configuré pour surveiller l'équilibre hydrique.

**15.** Moniteur selon l'une quelconque des revendications 8 à 14, où ledit moniteur est configuré pour surveiller le fonctionnement du myocarde.

**16.** Moniteur selon l'une quelconque des revendications 8 à 15, où ledit moniteur est configuré pour surveiller les changements de la fraction d'éjection.

Fig. 1 (Prior Art)

Echocardiogram

207
205
204
208
209
203
200
201

An echocardiogram uses sound waves to image the heart. Color doppler uses color to show blood flow

Transducer

Fig. 2a (Prior Art)

Fig. 2b (Prior Art)

EP 2 386 250 B1

Fig. 2c (Prior Art)

Fig. 2d (Prior Art)

Apical View Diastole  Apical View Systole

## Fig. 2e (Prior Art)

EP 2 386 250 B1

Fig. 2f (Prior Art)

260
VTI (average
blood flow
velocity)

261
LVOT area

VTI X LVOT area = Stroke Volume

Fig. 2g (Prior Art)

NICHE Monitor system 301

Fig. 3

RIBS 404

4th INTERCOSTAL SPACE  401

5th INTERCOSTAL SPACE 402

APEX OF HEART 403

Fig. 4a

Midsternal line  404

Midclavicular line  405

Anterior axillary line
406

Sternal notch

Angle of Louis

2nd Costal cartilage

Upper lobes

Middle lobe

Lower lobes

Fig. 4b

Fig. 5a

Fig. 5b

Fig. 5c

Perform echocardiogram examination (optional) — 602

Place ultrasound transducers and ECG electrodes on anterior chest — 604

Acquire ultrasound pulsed Doppler data — 606

Generate 3D velocity maps for myocardial tissue and for blood flow (optional) — 608

Determine temporal minima and maxima in velocity maps — 610

Acquire ECG image — 612

Calculate momentary and averaged values of E, E', S', A, A', E/E', E/A', LVOT, VTI, SV, CO, HR — 614

Display hemodynamic and cardiac function values in numerical and graphical form — 616

Detach transducers and ECG electrodes — 618

600

# Fig. 6

Fig. 7a

Fig. 7b

Fig. 8a

Fig. 8b

Fig. 9

1002 — Triangulate to determine position of maximum

↓

1004 — Reconstruct vector velocity

↓

1006 — Calculate angle between vector and apical transmitter

↓

1008

No ← Two or more points with same peak value?

↓ Yes

1010 — Repeat for all points

↓

1012 — Estimate position by taking median of positions

↓

Estimate vector by taking median of vector values — 1014

↓

Estimate magnitude as a median of magnitudes — 1016

↓

Check validity — 1018

↓

Associate values with anatomical location (optional) — 1020

↓

1000

Use angle to select transducers (optional) — 1022

↓

Fig. 10   Repeat for other transducer sets — 1024

Fig. 11

Fig. 12a

Fig.12b

## REFERENCES CITED IN THE DESCRIPTION

### Patent documents cited in the description

- EP 0747010 A, Schmiesing
- US 7054679 B, Hirsh
- US 7043292 B, Tarjan
- US 7022077 B, Mourad
- US 20040059220 A
- US 6875176 B, Mourad
- US 5409010 A, Beach and Overbeck
- US 5188106 A, Nappholz
- US 5139020 A, Koestner
- US 6261233 B, Kantorovich
- US 6730030 B, Palti

### Non-patent literature cited in the description

- **MIGUEL A. QUINONES ; CATHERINE M. OTTO ; MARCUS STODDARD ; ALAN WAGGONER ; WILLIAM A. ZOGHBI.** Recommendations for Quantification of Doppler Echocardiography: A Report From the Doppler Quantification Task Force of the Nomenclature and Standards Committee of the American Society of Echocardiography. *J Am Soc Echocardiogr,* 2002, vol. 15, 167-84
- *Circulation,* 2004, vol. 109, 2432-2439
- *JACC,* 2006, vol. 47, 1891-1900
- **ALAIN COMBES et al.** Tissue Doppler imaging estimation of pulmonary artery occlusion pressure in ICU patients. *Intensive Care Med,* 2004, vol. 30, 75-81
- **CHEUK-MAN YU ; JOHN E. SANDERSON ; THOMAS H. MARWICK ; JAE K. OH.** Tissue Doppler Imaging: A New Prognosticator for Cardiovascular Diseases. *J. Am. Coll. Cardiol.,* 2007, vol. 49, 1903-1914
- *JASE,* 2005, vol. 18, 148-154
- **L. E. KINSLER ; A. R. FREY ; A. B. COPPENS ; J. V. SANDERS.** Fundamentals of Acoustic. John wiley & Sons, Inc, 453-454